# EUROPEAN PATENT SPECIFICATION

(11) **EP 1 196 143 B1**
(45) Date of publication and mention of the grant of the patent: **01.03.2006**
(21) Application number: 00948142.5
(22) Date of filing: 24.07.2000
(51) Int. Cl.: A61K 9/00, A61K 9/50

(54) **USE OF AN ELECTRIC FIELD FOR SENSITISING RED BLOOD CELLS TO ULTRASOUND**
SENSIBILISIERUNG VON ROTEN BLUTKÖRPERCHEN GEGENÜBER ULTRASCHALL DURCH EINWIRKUNG EINES ELEKTRISCHEN FELDES
SENSIBILISATION DE GLOBULES ROUGES AUX ULTRASONS PAR APPLICATION D' UN CHAMP ELECTRIQUE

(30) Priority: 23.07.1999 GB 9917416; 30.07.1999 US 146556 P
(43) Date of publication of application: 17.04.2002
(73) Proprietor: Uutech Limited, Coleraine, County Londonderry N.Ireland (GB)
(72) Inventor: ROLLAN HARO, Ana Maria, Portstewart County Londonderry BT55 7GD (GB); MCHALE, Anthony Patrick, Portstewart, County Londonderry BT55 7GD (GB); CRAIG, Roger, Sandbach, Cheshire CW11 2XB (GB)
(74) Representative: Khoo, Chong-Yee
(86) International application number: PCT/GB2000/002848
(87) International publication number: WO 2001/007011

(56) References cited:
- EP-A- 0 367 475
- EP-A- 0 882 448
- EP-A- 0 898 889
- WO-A-97/33474
- US-A- 4 224 313
- US-A- 4 935 223
- US-A- 5 236 835
- T. WARD ET AL.: "the effects of electric fields on photosensitized erythrocytes: possible enhancement of photodynamic activation" CANCER LETTERS, vol. 106, 23 August 1996 (1996-08-23), pages 69-74, XP000857165 New York (US)
- CHEMICAL ABSTRACTS, vol. 90, no. 1, 1 January 1979 (1979-01-01) Columbus, Ohio, US; abstract no. 3815p, A.R. WILLIAMS ET AL.: "release of beta-thromboglobulin from human platelets by therapeutic intensities of ultrasound" page 373; column 2; XP002130796 & BR. J. HAEMATOL., vol. 40, no. 1, 1978, pages 133-142,
- Y. Mouneimne *a al.: 'Electro-insertion of xeno-glycophorin into the red blood cell membrane' BIOCHEMICAL AND BIOPHYSICAL RESEARCH COMMUNICATIONS; vol. 159, no. 1, 28 February 1989, pp. 34-40, XP002166099

## Description

The present invention relates to a method to enable the delivery of an agent to a target site.

In particular, the present invention relates to a method for enabling the delivering of an agent in a red blood cell loaded with the agent, which cell is sensitised to assist in agent release.

The delivery of a therapeutic agents to specific tissues is desirable typically to ensure that a sufficiently high dose of a given agent is delivered to a selected tissue. Moreover, it is often the case that the therapeutic agent, although advantageously having beneficial therapeutic effects on the diseased tissue, may have undesirable side effects on tissues that are not diseased. For example, in the treatment of certain types of disorders, such as cancer, it is necessary to use a high enough dose of a drug to kill the cancer cells without killing an unacceptable high number of normal cells. Thus, one of the major challenges of disease treatment is to identify ways of exploiting cellular drug delivery vehicles to incorporate and to selectively release agents at a desired target site.

It has been suggested that red blood cells may be exploited as active agent/drug delivery vehicles (DeLoach & Sprandel 1985, Bibliotheca Haematologica; Publ. Karger, Munich) as it is possible to incorporate agents into human red blood cells using a variety of techniques. An example of such a technique is the exploitation of osmotic shock and modifications thereof such as hypotonic shock and subsequent recovery of isotonicity and reverse hypotonic dialysis (Luque & Pinilla, 1993, Ind. Farmac. 8, 53-59).

An alternative method for loading drugs and active agents into red blood cells is electroporation. Using this process, the agent of interest are mixed with the live red blood cells in a buffer medium and short pulses of high electric fields are applied. The red blood cell membranes are transiently made porous and the agents of interest enter the cells. The electroporation process is advantageous as very high loading indices can be achieved within a very short time period (Flynn *et al.,* 1994, Cancer Letts., 82, 225-229).

When packaging/carrier/delivery systems such as red blood cells are used as *in vivo* delivery systems, they suffer from the drawback that the delivery function is dependent upon both an accumulation of the red blood cells and a breakdown of the red blood cell membrane in or at the relevant tissue/site. As a result, attempts have been made to incorporate sensitising agents into cell carriers in order to facilitate both the accumulation and/or release of an agent of interest at a target site.

By way of example, our co-pending UK Patent Applications 9816583.0 9826676.0 relate *inter alia* to the incorporation of a dye compound, such as a porphyrin, which renders a loaded red blood cell susceptible to laser light treatment at a target site. This phenomenon, known as photodynamic activation, is exploited in order to achieve accumulation of the carrier vehicle at the relevant site and to achieve load release at that site.

Alternative energy sources have been investigated as tools for inducing payload release from loaded and sensitised cells. By way of example, ultrasound irradiation has been investigated as an alternative to light induced photodynamic activation as it has a broader degree of focus and it penetrates more deeply into the body. However, although ultrasound irradiation has also been applied to effect red blood cell lysis *in vitro,* its use has been limited in that its effect is only significant at lower cell concentrations (1-6x10⁶ cells) (Brayman *et al*., 1996, Ultrasound in Med & Biol., 22: 497-514). Moreover, ultrasound is non-specific in effects, resulting in lysis of both loaded and endogenous red blood cells.

Recently, it has been found that certain dye compounds, in particular porphyrins, can achieve a cytopathogenic effect when the disease site is subjected to ultrasound irradiation. This technique is referred to as sonodynamic therapy and is discussed in WO98/52609. WO98/52609 teaches that ultrasound irradiation may be useful in treating disease but only when it is combined with an effective amount of an ultrasound-susceptibility modification agent such as a porphyrin.

We describe a method for selectively releasing an agent from a loaded red blood cell at a target site.

According to a first aspect of the present invention, we provide the use of an electric field under *in vitro* or *ex vivo* conditions for sensitising a red blood cell to ultrasound.

Preferably, the electric field has sufficient energy to electrosensitise the cell. More preferably, the red blood cell sensitised using electric field pulsing may be selectively disrupted using ultrasound.

According to a second aspect of the invention, we provide the use of ultrasound to disrupt an electrosensitised red blood cell, in which both the electrosensitisation and ultrasound disruption are carried out under *in vitro* or ex *vivo* conditions.

According to a third aspect of the invention, we provide an *in vitro* or *ex vivo* method of selectively disrupting a red blood cell, the method comprising the steps of: (a) electrosensitising said red blood cell; and (b) disrupting said red blood cell by subjecting said red blood cell to ultrasound. The method may further comprise loading the red blood cell with an agent under in *vitro* or *ex vivo* conditions.

Preferably, the electrosensitisation comprises the step of applying an electric pulse to a red blood cell. Preferably, the electric pulse is from about 0.1kVolts/cm to about 10 kVolts/cm under *in vitro* conditions. Preferably, the electric pulse is applied for between 1µs and 100 milliseconds.

A further step of loading the red blood cell with an agent may also be included.

The sensitisation of the red blood cell may precede the loading of the agent. Alternatively, the loading of the agent precedes the sensitisation of the red blood cell.

According to a fourth aspect of the invention, we provide an in *vitro* or *ex vivo* method for selectively releasing an agent from a red blood cell comprising the steps of: loading a red blood cell with an agent; electrosensitising the red blood cell; and causing the agent to be released from the electrosensitised red blood cell by applying ultrasound at a frequency and energy sufficient to cause disruption of the red blood cell but insufficient to cause disruption of unsensitised red blood cells.

According to a fifth aspect of the present invention, there is provided an *in vitro* or *ex vivo* method for providing a delivery vehicle suitable for delivery of an agent to a vertebrate, comprising the steps of: (a) loading a red blood cell with an agent; and, in a separate step, (b) exposing the red blood cell to an electric field to render it susceptible to disruption by ultrasound. We describe a method of introducing the sensitised red blood cell into the vertebrate; and causing the disruption of the sensitised red blood cell by treatment of the cell with ultrasound to release the agent at a target site.

According to a sixth aspect of the present invention, there is provided an *in vitro* or *ex vivo* method of electrosensitising a red blood cell to disruption by ultrasound, the method comprising the step of subjecting the red blood cell to an electric field of sufficient energy to sensitise the red blood cell so that it is susceptible to disruption by ultrasound.

The electrosensitised red blood cells may be loaded with agents either before, during or after the electrosensitisation procedure. In one aspect, therefore, the electrosensitisation procedure is effective to electroporate the cells, thus effecting simultaneous loading of a desired agent. Preferably, however, the electrosensitisation procedure is not effective to electroporate the cells, and the loading is thus carried out in a separate step either before or after the electrosensitisation procedure. Preferred methods for loading cells are set out below.

We describe an electrosensitised red blood cell which is preparable by subjecting a red blood cell to an electric field at an energy level which is not effective to electroporate the cell. We also describe such electrosensitised red blood cells which have been loaded with an agent using a process other than electroporation.

We further describe a kit comprising a red blood cell, an agent, packaging materials therefor and instructions for use, the use comprising the steps of: electrosensitising a red blood; loading the red blood cell with an agent; causing the agent to be released from the electrosensitised red blood cell by exposure to ultrasound at a frequency and energy effective to cause disruption of the sensitised red blood cell but insufficient to cause disruption of unsensitised red blood cells.

There is also described a kit comprising a red blood cell which is loaded with an agent, packaging materials therefor and instructions for use comprising the steps of: electrosensitising a red blood cell; and causing the agent to be released from the sensitised red blood cell by exposure to ultrasound at a frequency and energy effective to cause disruption of the sensitised red blood cell but insufficient to cause disruption of unsensitised red blood cells.

We further describe a kit comprising a loaded electrosensitised red blood and instructions for causing the agent to be released from the electrosensitised red blood cell by exposure to ultrasound at a frequency and energy effective to cause disruption of the sensitised red blood cell but insufficient to cause disruption of unsensitised red blood cells.

According to further aspects of the invention, we provide use of an electroporation apparatus under in *vitro* or *ex vivo* conditions to apply an electric field to a red blood cell for sensitising the red blood cell to disruption by ultrasound and use of an ultrasound apparatus under *in vitro* or *ex vivo* conditions for the disruption of an electrosensitised red blood cell.

In these and other aspects of the invention, the sensitisation and loading steps may be performed in any desired order, as appropriate.

In general, the electric field may have a strength from about 0.1k Volts/cm to about 10 kVolts/cm under *in vitro* conditions. Furthermore, the electric pulse may be applied for between 1 µs and 100 milliseconds.

In general, the ultrasound may be selected from the group consisting of: diagnostic ultrasound, therapeutic ultrasound and a combination of diagnostic and therapeutic ultrasound. The ultrasound may be applied at a power level of from about 0.05W/cm² to about 100W/cm².

The red blood cell may be loaded with an agent using a procedure selected from a group consisting of electroporation, sonoporation, microinjection, membrane intercalation, microparticle bombardment, lipid-mediated transfection, viral infection, osmosis, osmotic pulsing, diffusion, endocytosis, and crosslinking to a red blood cell surface component.

The agent may be selected from a group consisting of a biologically active molecule, a protein, a polypeptide, a peptide, a nucleic acid, a virus, a virus-like particle, a nucleotide, a ribonucleotide, a deoxyribonucleotide, a modified deoxyribonucleotide, a heteroduplex, a nanoparticle, a synthetic analogue of a nucleotide, a synthetic analogue of a ribonucleotide, a modified nucleotide, a modified ribonucleotide, an amino acid, an amino acid analogue, a modified amino acid, a modified amino acid analogue, a steroid, a proteoglycan, a lipid, a carbohydrate, and mixtures, fusions, combinations or conjugates of the above. The agent may be conjugated to, fused to, mixed with or combined with an imaging agent, and the red blood cell may furthermore be PEGylated.

### Brief Description of the Figures

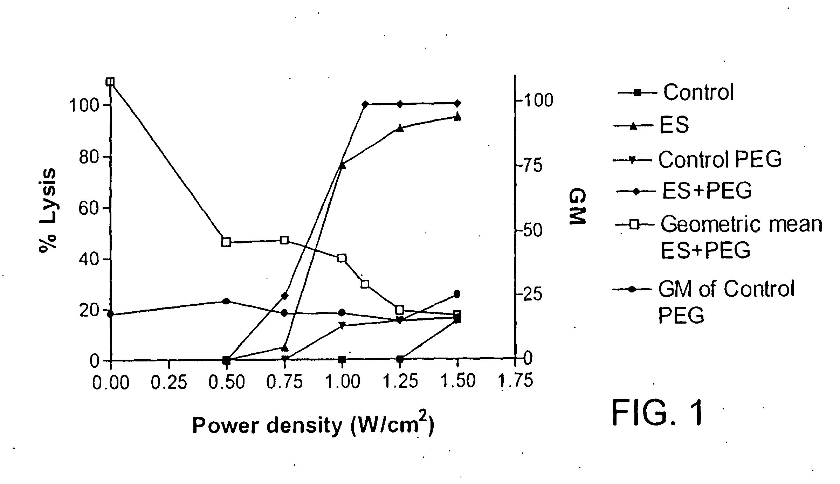

Figure 1 shows the effect of ultrasound power density on control normal (■), electrosensitised normal (▲), control PEG-treated (▼), and electro-sensitised PEG-treated human red blood cells in PBS/Mg/glucose. X-axis: power density (W/cm²); left hand Y-axis: % lysis. The geometric mean (right-hand Y-axis) of fluorescence from populations of PEG-treated control (●) and electro-sensitised PEG-treated human red blood cells exposed to each power density and determined using flow cytometry are also plotted.

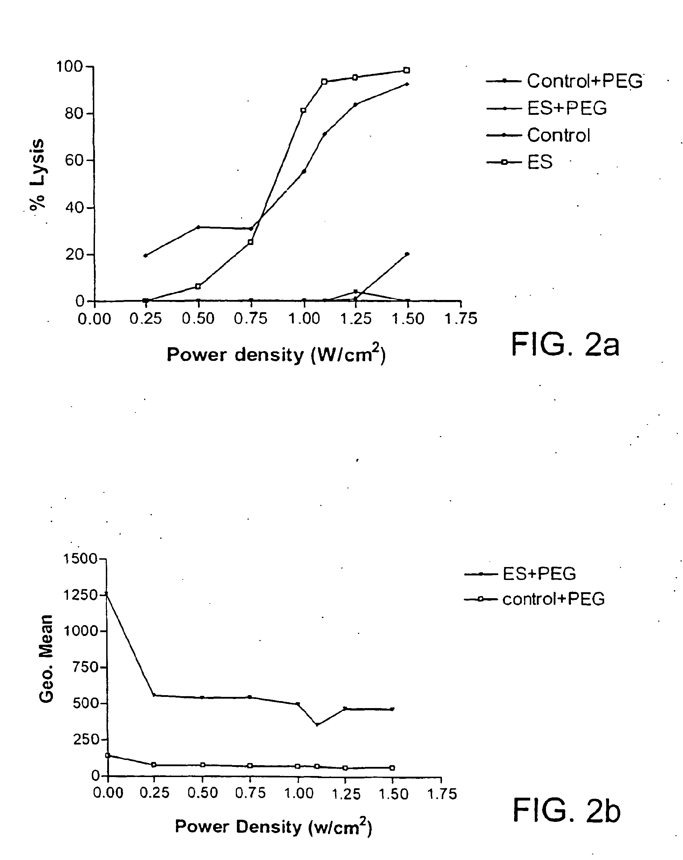

Figure 2A shows the effect of ultrasound power density on control normal (●), control PEG-treate (▼), electro-sensitised (□) and electro-sensitised, PEG-treated (◆) human red blood cells in autologous plasma. X-axis: power density (W/cm²); Y-axis: % lysis.
Figure 2B shows the geometric mean of fluorescence from populations of PEG-treated control (□) and electro-sensitised PEG-treated (▼) human red blood cells exposed to each power density and determined using flow cytometry. X-axis: power density (W/cm²); Y-axis: geometric mean.

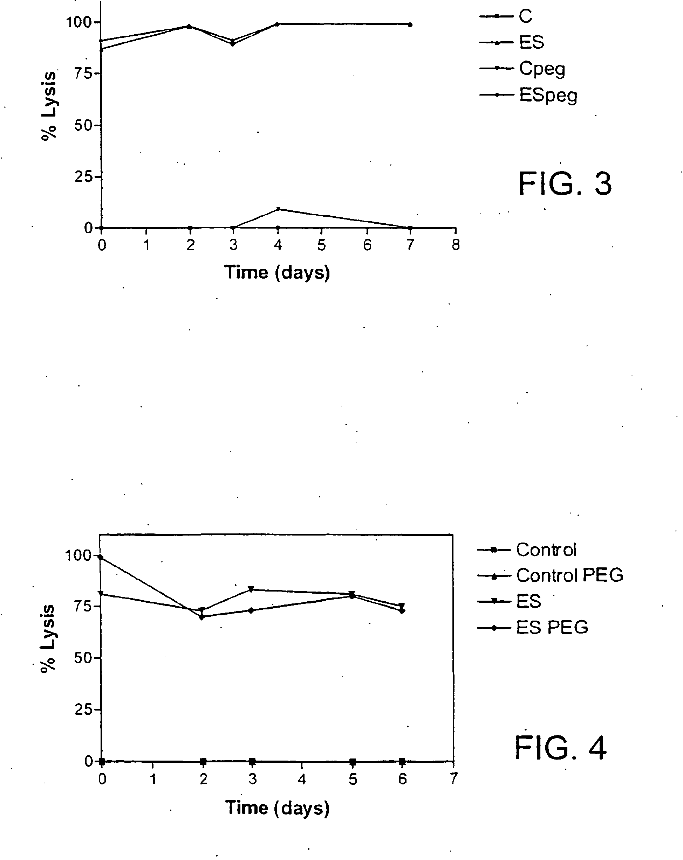

Figure 3 shows the effect of ultrasound (1.25W/cm², 30 seconds) on control normal (■), control PEG-treated (▼), electro-sensitised normal (▲) and electro-sensitised PEG-(◆) human red blood cells which had been stored for the indicated times at 4°C in PBS/Mg/glucose. X-axis: time (days); Y-axis: % lysis.
Figure 4 shows the effect of ultrasound (1.25W/cm²) on control normal (■), control PEG-treated (▲), electro-sensitised normal (▼) and electro-sensitised PEG-treated (◆) human red blood cells stored for the indicated times at 4°C in autologous plasma. X-axis: time (days); Y-axis: % lysis.

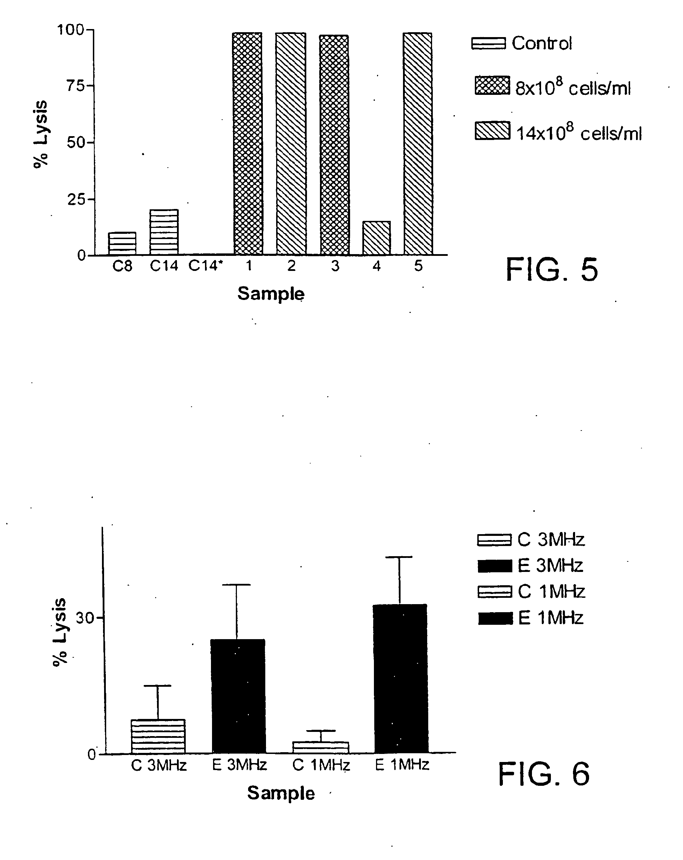

Figure 5 shows the effect of cell concentration on electro-sensitisation to ULTRASOUND. Cells were (i) electro-sensitised at 8x10⁸ cells/ml, re-sealed in PBS/Mg, stored in PBS/Mg/glucose for 1 and 3h and finally subjected to ULTRASOUND (1.5W/cm², 3MHz, 5 min.) (Samples 1 and 3, respectively). Alternatively (ii) cells were electro-sensitised at 14x10⁸ cells/ml and treated in a similar manner before treatment with ULTRASOUND (Samples 2 and 4, respectively). Finally (iii) Sample 5 consisted of cells which were electro-sensitised at 8x10⁸ cells/ml, re-sealed as described above, pooled to 14x10⁸ cells/ml, stored for 3h and finally treated with ULTRASOUND. Control samples, C8, C14 and C14* consisted of cells treated in a similar manner to the three sets of cells described above (i, ii, iii, respectively) excluding electro-sensitisation. X-axis: sample number; Y-axis: % lysis.
Figure 6 shows the response of electro-sensitised and normal human red blood cells in a soft tissue mimicking phantom system following exposure to 5 min. ULTRASOUND at 1.5W/cm² and at 3 and 1MHz. Cell samples were placed at an average distance of 1cm from the scanning surface (ULTRASOUND head surface) during treatments. Samples were then retrieved from the system and cell counts were determined using a hemocytometer. X-axis: sample number; Y-axis: % lysis.

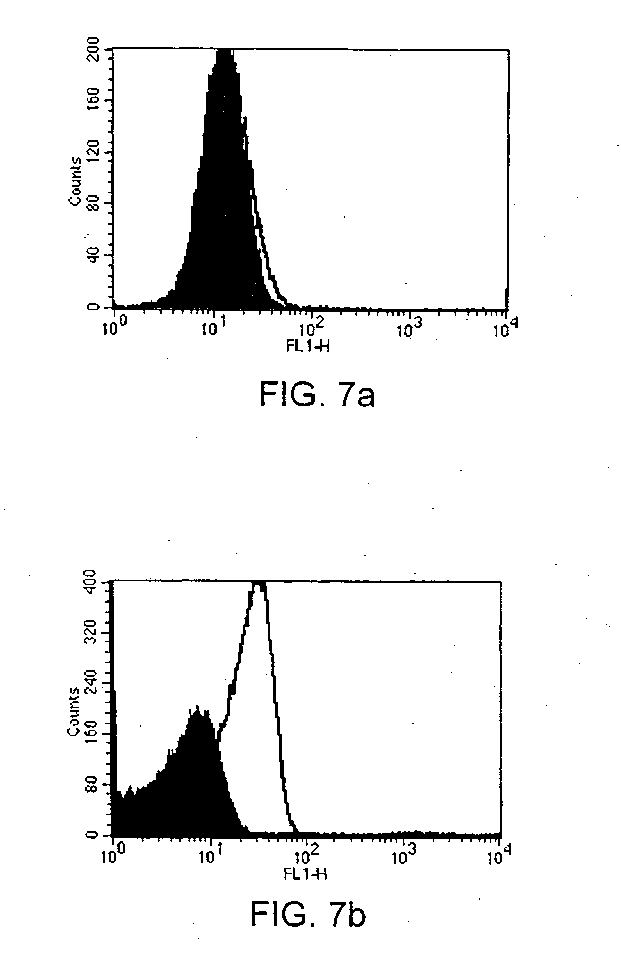

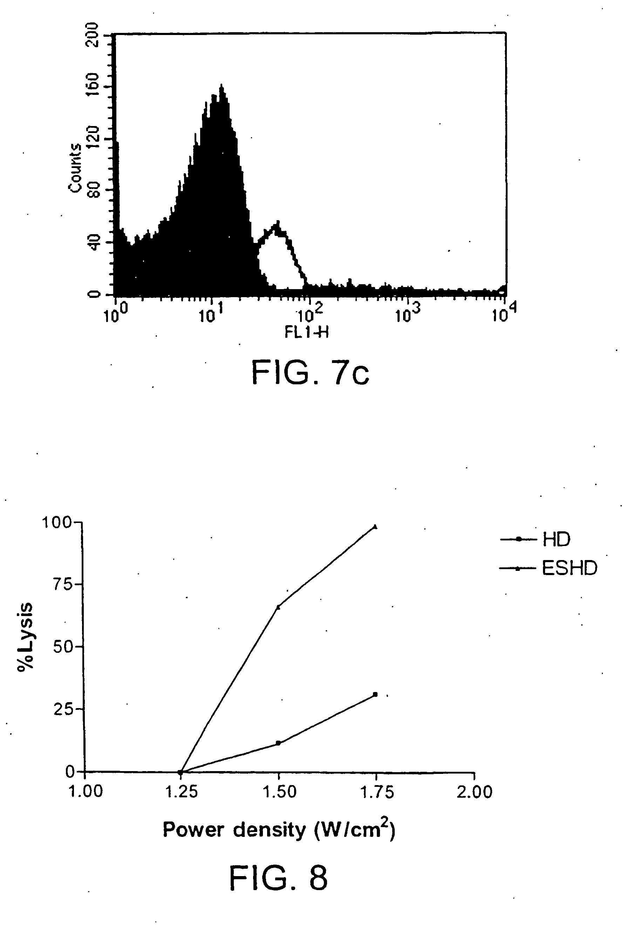

Figure 7 shows a flow cytometer analysis of cells subjected to loading by electroporation at varying electric field strengths. The filled traces represent cells which have not been electroporated, but exposed to antibody; these traces are overlayed with an open trace, representing cells which have been subjected to electroporation, with the antibody. The conditions are as follows: panel (a) 5x10⁷ RBC + 0.5mg/ml antibody, pulsing twice at 1.45 KV 1 µF in PBS at 4 degrees C; panel (b) 3.5x10⁷ RBC + 0.25mg/ml antibody, pulsing at 0.3 KV 10 µF, 1.45 KV 1 µF and 0.3 KV 10 µF in PBS at 4 degrees C; pane (c) 4.5x10⁷ RBC + 0.25mg/ml antibody, pulsing at 0.3 KV 10 µF, 1.45 KV 1 µF and 0.3 KV 10 µF in PBSucrose at 4 degrees C. X-axis: FLH-1; Y-axis: counts.
Figure 8 shows the effects of ultrasound on (i) cells treated with hypotonic dialysis (HD) (■) and (ii) those treated with the hypotonic dialysis protocol, rested overnight at 4°C and electro-sensitised (▲). X-axis: power density (W/cm²); Y-axis: % lysis.

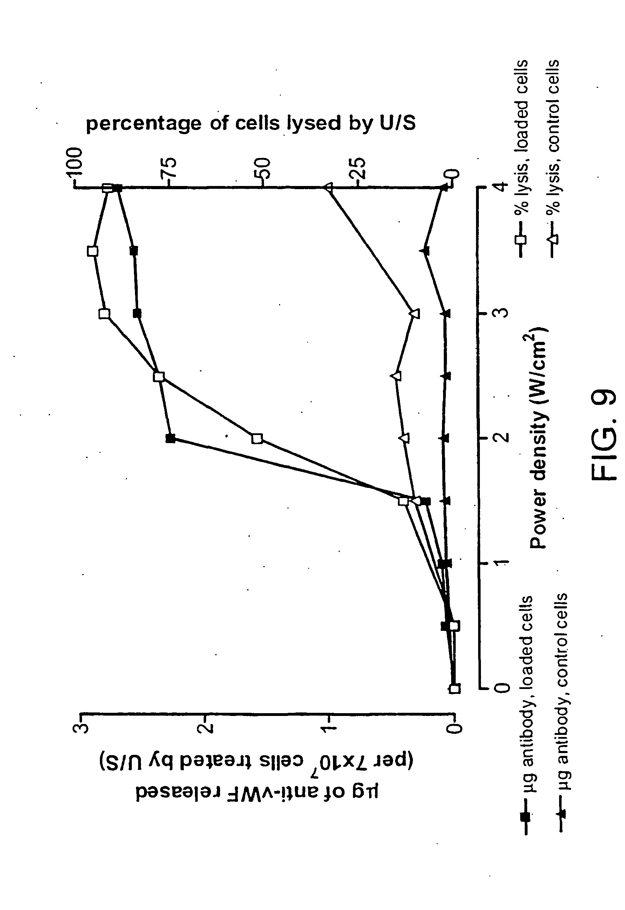

Figure 9 is a graph showing the ultrasound-mediated release of antibody from the erythrocyte vehicle. X-axis: power density (W/cm²), left hand Y-axis: µg of anti-vWF released (per 7x10⁷ cells treated by ultrasound); right hand Y-axis: percentage of cells lysed by ultrasound. Filled squares represent µg antibody, loaded cells; filled triangles represent µg antibody, control cells; open squares represent % lysis, loaded cells; open triangles represent % lysis, control cells.

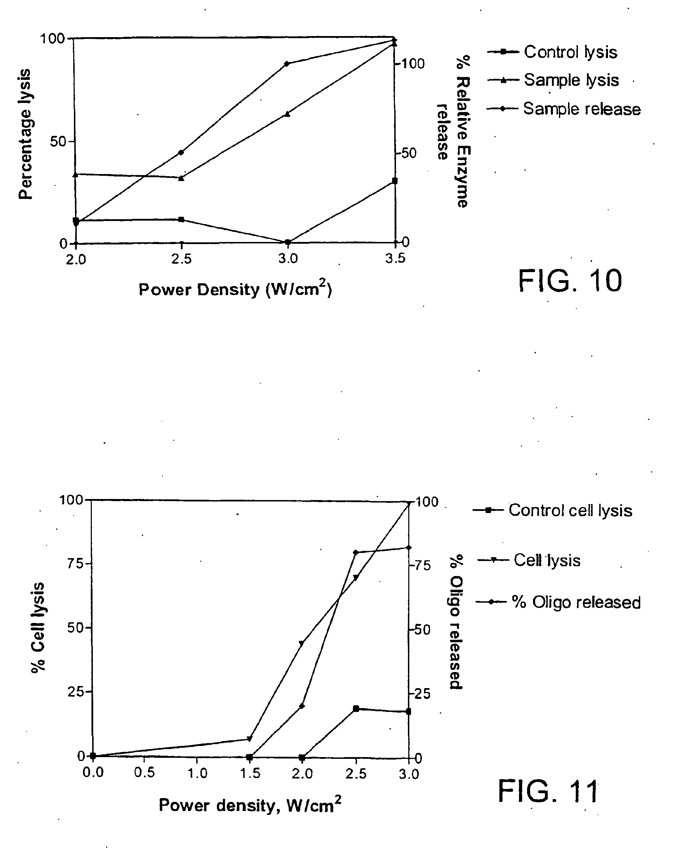

Figure 10 is a graph showing ultrasound mediated release of β-galactosidase from the erythrocyte vehicle. X-axis: power density (W/cm²), left hand Y-axis: percentage lysis; right hand Y-axis: % relative enzyme release. Filled squares represent control lysis, filled triangles represent sample lysis and filled diamonds represent sample release (i.e., release of enzyme).
Figure 11 is a graph showing ultrasound-mediated release of oligonucleotide from the erythrocyte vehicle. X-axis: power density (W/cm²), left hand Y-axis: percentage cell lysis; right hand Y-axis: % oligo release. Filled squares represent control cell lysis, filled triangles represent cell lysis and filled diamonds represent % oligo released.

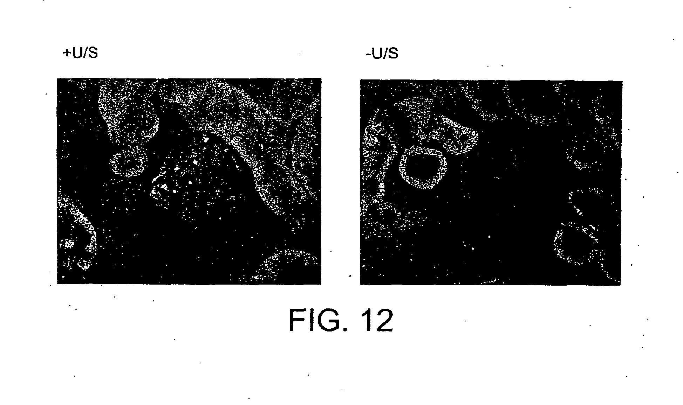

Figure 12 shows ultrasound-mediated release of anti von Willebrand factor antibody from sensitised human erythrocytes in perfused rat kidney.

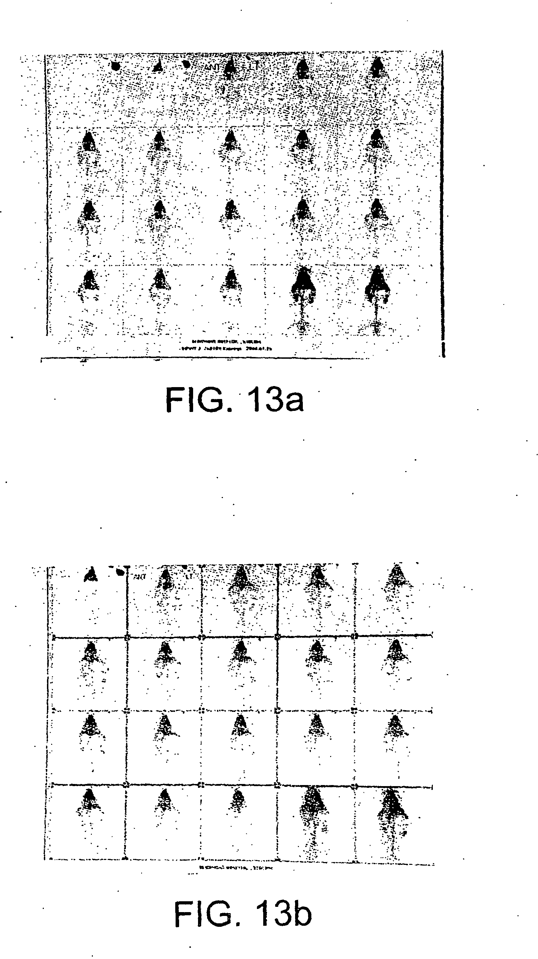

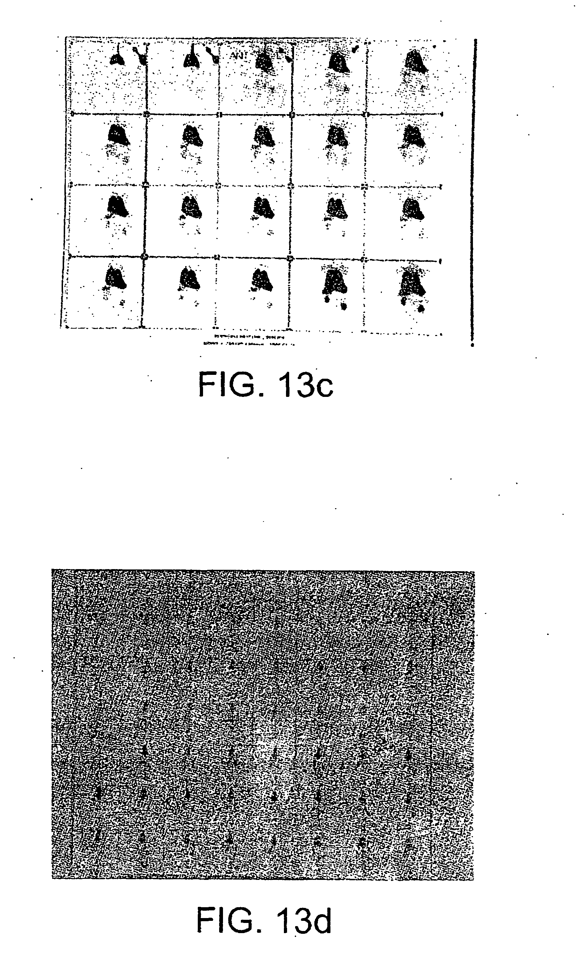

Figure 13 shows gamma camera imaging of ⁹⁹Tc labelled electrosensitised (A), normal (B), glutaraldehyde-treated (C) and PEGylated (D) rabbit erythrocytes during circulation in a host rabbit. Images were captured over a 20 min. period at intervals. Figures 13A, B and C: first row 10", 30", 1', 1'30", 2'; second row 2'30", 3', 4', 5', 6'; third row 7', 8', 9'30", 11', 12'30"; fourth row 14', 15'30", 16', 18'30", 20'. Figure 13D: row 1 10s, 20s, 30s, 40s, 50s, 1', 70s, 80s; row 2 90s, 100s, 110s, 2', 130s, 140s, 150s, 160s; row 3 170s. 3', 3'20s, 3'40s, 4', 4'20s, 4'40s, 5'; row 4 5'20s, 5'40s, 6', 6'20s, 6'40s, 7', 7'20s, 7'40s; row 5 8', 8.5', 9', 9.5', 10', 10.5', 11', 11.5'; row 6 12', 12.5', 13', 13.5', 14', 14.5', 15', 15.5'; row 7 16', 16.5', 17', 17.5', 18', 18.5', 19', 19.5'.

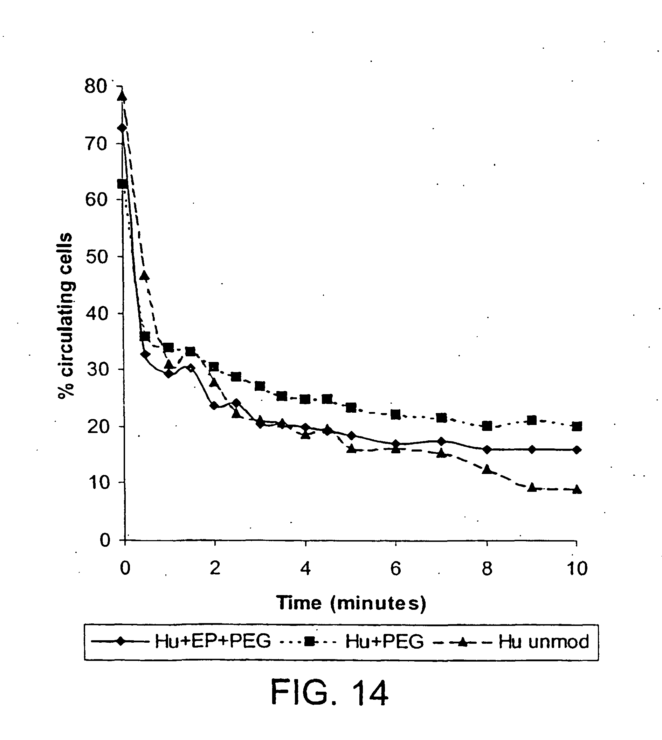

Figure 14 shows clearance of ⁹⁹Tc-labelled normal (▲), PEGylated normal (■) and PEGylated electrosensitised (◆) human erythrocytes during circulation in a recipient rabbit. X-axis: Time (minutes), Y-axis: %circulating cells.

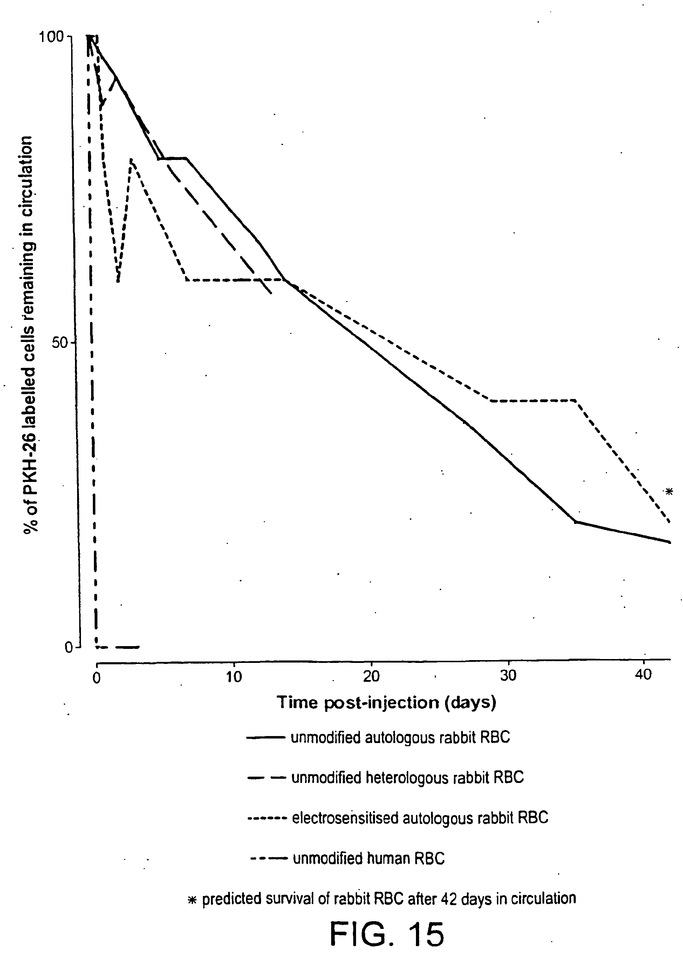

Figure 15 shows *in vivo* survival of PKH-26 labelled, normal autologous, normal heterologous and electrosensitised rabbit erythrocytes in recipient rabbits. X-axis: time post-injection (days); Y-axis % of PKH-26 labelled cells remaining in circulation. Normal human erythrocytes are included as a control for sequestration by the reticulo-endothelial system. ― (continuous) unmodified autologous rabbit RBC; ― ― ―unmodified heterologous rabbit RBC; --- electrosensitised autologous rabbit RBC; ― -- ― -- unmodifiedl\uman RBC; * predicted survival of rabbit RBC after 42 days in circulation.

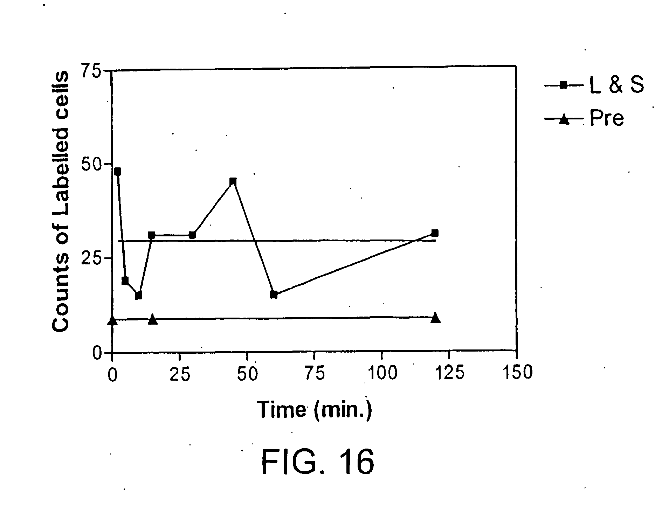

Figure 16 shows survival of PKH-26-labelled antibody-loaded and sensitised rabbit *erythrocytes in vivo.* X-axis: time (min); Y-axis: counts of labelled cells. The base line indicated by ▲ is for reference purposes and indicates the level of counts detectable prior to introduction of the labelled erythrocytes. Filled squares represent loaded and sensitised rabbit erythocytes.

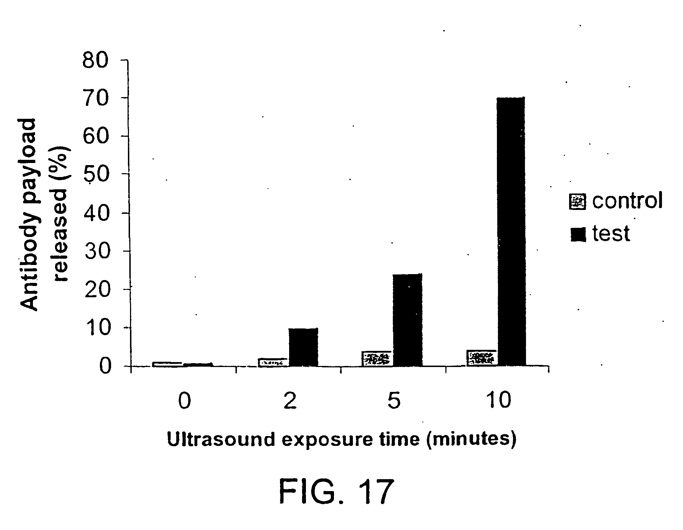

Figure 17 shows ultrasound-mediated release of antibody payload (anti-von Willebrand factor antibody) from loaded and sensitised human cells diluted in normal human cells at 40% hematocrit. Continuous wave ultrasound at 5W/cm² is used. X-axis: ultrasound exposure time (minutes); Y-axis: antibody payload released (%). The target cells were circulated through a system in which the temperature was maintained at 37°C and the flow rate during exposure was 14.5ml/min. Gray bars: control; black bars: test.

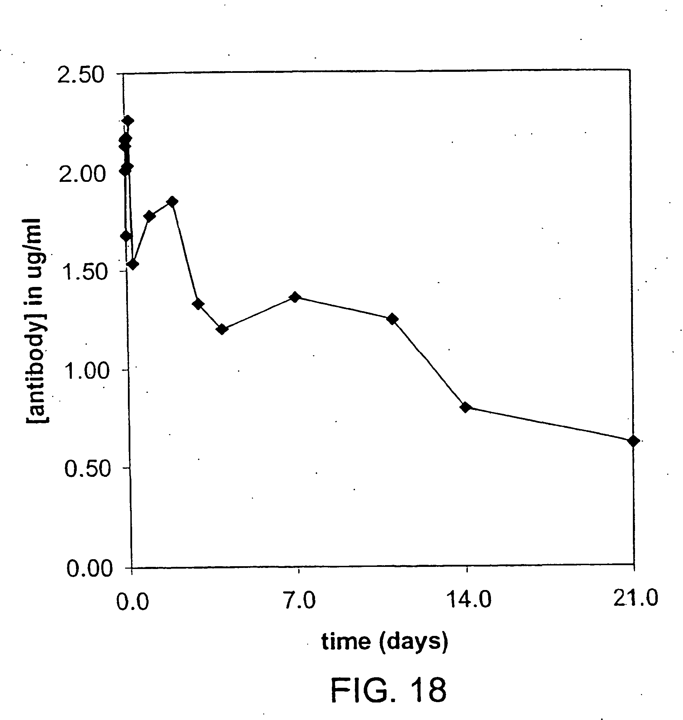

Figure 18 shows clearance of rabbit-anti human IgG from rabbit circulation (n=3) as measured by ELISA. X-axis: time(days); Y-axis: antibody concentration in micrograms/ml.

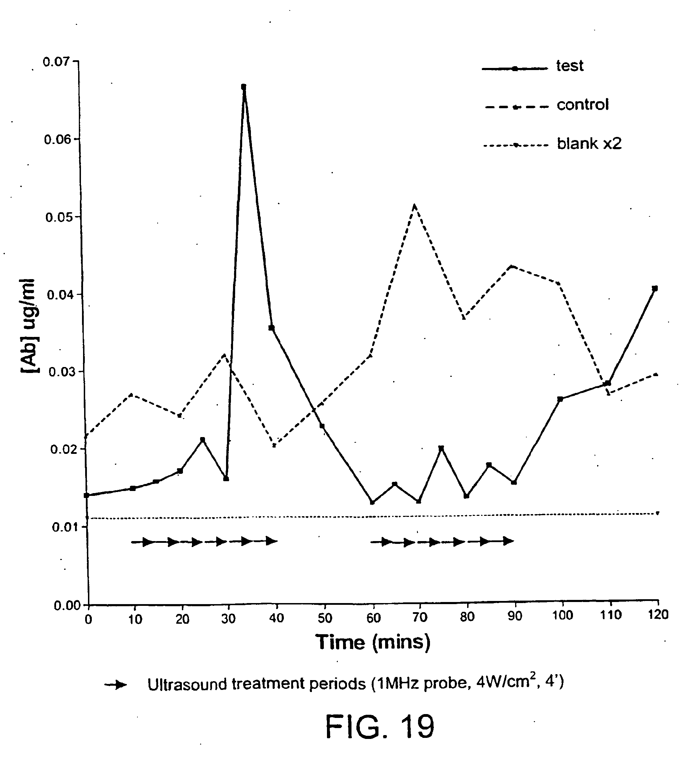

Figure 19 shows ultrasound-mediated release of rabbit anti-human IgG from loaded and sensitised rabbit erythrocytes following exposure to ultrasound during circulation *in vivo.* X-axis: time (mins); Y-axis: antibody concentration in micrograms/ml. Filled squares (continuous ―): test; filled upright triangles (black dashed -): control; filled inverted triangles (grey dashed -) represent pre-injection signal x 2; right-pointing arrows: ultrasound treatment periods (1MHz probe, 4W/cm², 4').

The present invention demonstrates the highly surprising findings that:
(i) exposure of red blood cells to electrosensitisation induces a hyper-sensitivity to ultrasound.
(ii) exposure of red blood cells to electrosensitisation induces a hyper-sensitivity to ultrasound without the addition of chemical agents.
(iii) exposure of red blood cells to electrosensitisation induces a hyper-sensitivity to ultrasound and allows the selective lysis of destabilised red blood cells with ultrasound with little or no effect on normal red blood cells under *in vitro* and *in vivo* conditions.
(iv) the present invention allows for the targeted delivery of an agent to a tissue of interest in a vertebrate using sensitised red blood cells which have no particular affinity for the target tissue. This is of particular importance where the target tissue is of a type which is widely distributed throughout the body (for example, skeletal muscle).

### Electrosensitisation

The term "electrosensitisation" encompasses the destabilisation of cells without causing fatal damage to the cells. According to this method, a momentary exposure of a cell to a high electric field results in membrane destabilisation. The strength of the electric field is adjusted up or down depending upon the resilience or fragility, respectively, of the cells being loaded and the ionic strength of the medium in which the cells are suspended.

Electrosensitisation typically involves the use of electric fields which do not possess sufficient energy to electroporate the cells. Electroporation, which facilitates the passage of agents into the cell without significant loss of cellular contents or cell viability, is well known in the art, and apart from the energy levels involved is similar to electrosensitisation. Indeed, cells which are electroporated become electrosensitised. However, electrosensitisation may be carried out at energy levels which are insufficient to electroporate the cell and permit the passage of substances through the cell wall. Thus, the invention encompasses the use of an electric field for sensitising a red blood cell to ultrasound.

Electroporation has been used in both *in vitro* and *in vivo* procedures to introduce foreign material into living cells. With *in vitro* applications, a sample of live cells is first mixed with the agent of interest and placed between electrodes such as parallel plates. Then, the electrodes apply an electrical field to the cell/implant mixture. Examples of systems that perform *in vitro* electroporation include the Electro Cell Manipulator ECM600 product, and the Electro Square Porator T820, both made by the BTX Division of Genetronics, Inc (see US Patent No 5869326).

These known electroporation techniques (both *in vitro* and *in vivo)* function by applying a brief high voltage pulse to electrodes positioned around the treatment region. The electric field generated between the electrodes causes the cell membranes to temporarily become porous, whereupon molecules of the agent of interest enter the cells. In known electroporation applications, this electric field comprises a single square wave pulse on the order of 1000 V/cm, of about 100 µs duration. Such a pulse may be generated, for example, in known applications of the Electro Square Porator T820.

Electrosensitisation may be performed in a manner substantially identical to the procedure followed for electroporation, with the exception that lower electric field strengths may be used, as set forth below.

In a preferred aspect of the present invention, the electric field has a strength of from about 0.1 kVolts /cm to about 10 kVolts/cm under *in vitro* conditions.

Preferably the electric field has a strength of from about 1.5 kVolts/cm to about 4.0 kVolts/cm under *in vitro* conditions.

Preferably the electric field has a strength of from about 0.1kVolts /cm to about 10 kVolts/cm under *in vivo* conditions (see WO 97/49450).

Preferably the application of the electric field comprises multiple pulses.

Preferably the application of the electric field comprises sequential pulses (see Table 1).

Preferably the application of the electric field comprises double pulses.

In preferred embodiments, the electric pulse is applied for between 1µs and 100 milliseconds.

Preferably the electric pulse is delivered as an exponential wave form.

Preferably the electric pulse is delivered as a square wave form.

Preferably the electric pulse is delivered as a modulated wave form.

As used herein, the term "electric pulse" includes one or more pulses at variable capacitance and voltage and including exponential and/or square wave and/or modulated wave forms.

Other electroporation procedures and methods employing electroporation devices are widely used in cell culture, and appropriate instrumentation is well known in the art.

### Loading

As used herein, the term "loading' refers to a red blood cell which comprises at least one agent. The agent may be loaded by becoming internalised by, affixed to the surface of, or anchored into the plasma membrane of a red blood cell. Where the agent is affixed or anchored to the plasma membrane, loading may be achieved by cross-linking the agent to any cell surface molecule. Alternatively, the agent may be conjugated to or fused with an antibody specific for a cell surface molecule.

Loading of a red blood cell with more than one agent may be performed such that the agents are loaded individually (in sequence) or together (simultaneously or concurrently) and/or prior to, simultaneous with, sequential to or separate from, the "sensitising" procedure. The agents may be first admixed at the time of contact with the red blood cells or prior to that time.

The red blood cells may be loaded either prior to, simultaneously with, or after the sensitisation procedure. In one embodiment the red blood cells may be pre-loaded with the desired agent, and subsequently electrosensitised. In this embodiment, the loading may be performed by any desired technique. If they are loaded and sensitised substantially simultaneously, they may be loaded and sensitised by the same technique. Alternatively, the red blood cells may be sensitised and subsequently loaded. By way of example, the red blood cell may be sensitised by electrosensitisation, and loaded using osmotic shock or using electroporation. If more than one agent is employed, the same or a different technique may be used to load the second agent into the red blood cell. In general, if loading is subsequent to sensitisation, two or more agents can be loaded in any order. If loading is simultaneous, two or more agents can be admixed prior to contact with the red blood cells or can be added separately, prior to or after the application of the loading procedure mediates uptake of the agents by the cell.

As used herein, the term "substantially simultaneous" means that the site and time of loading and sensitisation are such that the loading and sensitisation are achieved at approximately the same time.

Preferably the red blood cells are sensitised and loaded (in any order) *in vitro* or *ex vivo.*

Preferably the loading is performed by a procedure selected from the group consisting of electroporation, sonoporation, microinjection, calcium precipitation, membrane intercalation, microparticle bombardment, lipid-mediated transfection, viral infection, osmosis, osmotic pulsing, osmotic shock, diffusion, endocytosis, phagocytosis, crosslinking to a red blood cell surface component, chemical crosslinking, mechanical perforation/restoration of the plasma membrane by shearing, single-cell injection or a combination thereof. Sonoporation as a method for loading an agent into a cell is disclosed in, for example, Miller *et al* (1998), *Ulirusonics* 36, 947-952.

In a preferred aspect, the loading procedure is carried out by iontophoresis.

lontophoresis uses electrical current to activate and to modulate the diffusion of a charged molecule across a biological membrane, such as the skin, in a manner similar to passive diffusion under a concentration gradient, but at a facilitated rate. In general, iontophoresis technology uses an electrical potential or current across a semipermeable barrier. By way of example, delivery of heparin molecules to patients has been shown using iontophoresis, a technique which uses low current (d.c.) to drive charged species into the arterial wall. The iontophoresis technology and references relating thereto is disclosed in WO 97/49450.

In a further preferred aspect, loading is carried out by an osmotic shock procedure. in more detail, the "osmotic shock" mechanism is taught in U.S. Pat. No. 4,478,824. That method involves incubating a packed red blood cell fraction in a solution containing a compound (such as dimethyl sulphoxide (DMSO) or glycerol) which readily diffuses into and out of cells. rapidly creating a transmembrane osmotic gradient by diluting the suspension of red blood cell in the solution with a near-isotonic aqueous medium. This medium contains an anionic agent to be introduced (such as a phosphorylated inositol) which may be an allosteric effector of haemoglobin, thereby causing diffusion of water into the cells with consequent swelling thereof and increase in permeability of the outer membranes of the cells. This increase in permeability is maintained for a period of time sufficient only to permit transport of the anionic agent into the cells and diffusion of the readily-diffusing compound out of the cells. This method is of limited effectiveness where the desired agent to be loaded into cells is not anionic, or is anionic or polyanionic but is not present in the near-isotonic aqueous medium in sufficient concentration to cause the needed increase in cell permeability without cell destruction.

U.S. Patent No. 4,931,276 and WO 91/16080 disclose methods of loading red blood cells with selected agents using an osmotic shock technique. Therefore, these techniques can be used to enable loading of red blood cells as described in this document. In U.S. Patent No. 4,931,276 a modified osmotic shock technique is provided.

Effective agents which may advantageously be loaded into red blood cells using the modified method provided in U.S. Patent No. 4,931,276 include peptides, purine analogues, pyrimidine analogues, chemotherapeutic agents and antibiotic agents. These agents frequently present drug delivery problems. Specific compounds include but are not limited to tryptophan, phenylalanine and other water-soluble amino acid compounds. Several derivatives of the unnatural analogues of the nucleic acid bases adenine, guanine, cytosine and thymine are well known as useful therapeutic agents, e.g., 6-mercaptopurine (6MP) and azathioprine, which are commonly used as immunosuppressants and inhibitors of malignant cell growth, and azidothymidine (AZT) and analogues thereof which are useful as anti-viral agents, particularly in the treatment of AIDS. It has been shown that the action of these unnatural base derivatives is dependent on intra-cellular conversion thereof to phosphorylated forms (Chan *et al.,* 1987, Pharmacotherapy, 7: 165;14 177; also Mitsuya *et al.,* 1986, Proc. Natl. Acad. Sci. U.S.A., 83: 1911-1915).

An alternative osmotic shock procedure is described in U.S. Patent No. 4,931,276.

In an advantageous aspect, loading is carried out by a microparticle bombardment procedure.

Microparticle bombardment entails coating gold particles with the agent to be loaded, dusting the particles onto a 22 calibre bullet, and firing the bullet into a restraining shield made of a bullet-proof material and having a hole smaller than the diameter of the bullet, such that the gold particles continue in motion toward cells *in vitro* and, upon contacting these cells, perforate them and deliver the payload to the cell cytoplasm.

It will be appreciated by one skilled in the art that combinations of methods may be used to facilitate the loading of a red blood cell with agents of interest. Likewise, it will be appreciated that a first and second agent, may be loaded concurrently or sequentially, in either order, into a red blood cell in any method as described.

As would be apparent to one of skill in the art, any one or more of the above techniques can be used to load red blood cells either prior to, simultaneously with, separate from or in sequence to the sensitisation procedure. For example, U.S. Patent No. 4,224.313 discloses a process for preparing a mass of loaded cells suspended in a solution by increasing the permeability of the cell membranes by osmotic pressure or an electric field, or both, loading agents by passage from a solution through the membranes of increased permeability, restoring the original permeability by sealing the membranes by regeneration effect, and separating the cells from the solution in which they were suspended. In that procedure, the agents in solution which are to be loaded include i) a pharmaceutical substance which reacts chemically or physically with substances in the extracellular milieu and which, when loaded into the cell, would prematurely destroy the cell membranes, and ii) at least one blood-compatible sugar and protein capable of providing hydrogen bridge bonding- or of entering into covalent bonds with the pharmaceutical substance, thereby inhibiting the reaction of the pharmaceutical substance with the cell membranes.

### Selective Release using Ultrasound

According to the methods described here, agents which are loaded into a red blood cell are released from the red blood cell and into their surroundings, in this case at or into the target site, tissue or cell, by the application of ultrasound directed at a target site, tissue and/or cell.

As used herein. the term "ultrasound" refers to a form of energy which consists of mechanical vibrations the frequencies of which are so high they are above the range of human hearing. Lower frequency limit of the ultrasonic spectrum may generally be taken as about 20kHz. Most diagnostic applications of ultrasound employ frequencies in the range 1 and 15MHz. (From Ultrasonics in clinical diagnosis. Edited by PNT Wells, 2nd. Edition, Publ. Churchill Livingstone [Edinburgh, London & NY, 1977]. The term "ultrasound" includes diagnostic, therapeutic and focused ultrasound. Diagnostic ultrasound refers to an ultrasound energy source in a range up to about 100mW/cm² (FDA recommendation). Therapeutic ultrasound refers to an ultrasound energy source in a range up to about 3-4W/cm² (WHO recommendation).

Focused ultrasound (FUS) allows thermal energy to be delivered without an invasive probe (see Morocz *et al* 1998 Journal of Magnetic Resonance Imaging Vol.8, No.1, pp.136-142. Another form of focused ultrasound is high intensity focused ultrasound (HIFU) which is reviewed by Moussatov *et al* in ULTRASONICS 1998 Vol.36, No.8, pp.893-900 and TranHuuHue *et al* in ACUSTICA, 1997, Vol.83, No.6, pp.1103-1106.

Preferably, a combination of diagnostic ultrasound and a therapeutic ultrasound is employed.

Preferably the ultrasound is applied to a target cell or target tissue with sufficient strength to disrupt loaded and sensitised red blood cells but without damaging the target tissue or surrounding tissues. In the context of the present document, the term "damage or damaging" does not include a transient permeabilisation of the target site by the ultrasound energy source. Such a permeabilisation may facilitate uptake of the released payload at the target site.

Preferably the exposure to an ultrasound energy source is at a power density of from about 0.05 to about 100 Wcm⁻².

Even more preferably, the exposure to an ultrasound energy source is at a power density of from about 1 to about 15 Wcm⁻².

Preferably the exposure to an ultrasound energy source is at a frequency of from about 0.015 to about 10.0 MHz.

Preferably the exposure to an ultrasound energy source is at a frequency of from about 0.02 to about 5.0 Mhz.

Preferably the exposure is for periods of from about 10 milliseconds to about 60 minutes.

Preferably the exposure is for periods of from about 1 second to about 5 minutes.

Particularly preferably the patient is exposed to an ultrasound energy source at an acoustic power density of from about 0.05Wcm⁻² to about 10Wcm⁻² with a frequency ranging from about 0.015 to about 10MHz (see WO 98/52609). However, alternatives are also possible, for example, exposure to an ultrasound energy source at an acoustic power density of above 100Wcm⁻², but for reduced periods of time, for example, 1000Wcm⁻² for periods in the millisecond range or less.

Use of ultrasound is advantageous as, like light, it can be focused accurately on a target. Moreover, ultrasound is advantageous as it has a broader degree of three-dimensional focus than a light energy source and is better suited to whole-tissue penetration (such as but not limited to a lobe of the liver) or whole organ (such as but not limited to the entire liver or an entire muscle, such as the heart) delivery of agents according to the present invention, in addition, ultrasound may induce a transient permeabilisation of the target site so that uptake of a released payload is facilitated at the target site. Another important advantage is that ultrasound is a non-invasive stimulus which is used in a wide variety of diagnostic and therapeutic applications. By way of example, ultrasound is well known in medical imaging techniques and, additionally, in orthopaedic therapy. Furthermore, instruments suitable for the application of ultrasound to a subject vertebrate are widely available and their use is well known in the art.

In the methods described here, release of the agent is effected by exposure of red blood cells either in *vitro* or *ex-vivo* to an effective amount of a diagnostic ultrasound energy source or a therapeutic ultrasound energy source as described in US Patent No. 5558092 and WO94/28873. The agent which is released from a red blood cell may be referred to as the "payload" of that cell. The term "payload" does not refer to the naturally-occurring contents of a red blood cell.

Preferably the agent is released from the red blood cell by treatment of a target site, tissue or cell with ultrasound.

The selective release of the agent at the target site can be determined by observing a) the amount which has been released at the target site, tissue or cell and b) its effect on the target site, tissue or cell, the latter determining whether its delivery should increase, decrease or be discontinued.

### Blood Cells

In one embodiment, the red blood cells which may be loaded and administered to a vertebrate are ideally obtained from the intended recipient individual prior to the procedure so as to ensure complete immunocompatibility. Alternatively, cells are obtained from a second individual of the same species as the recipient; in such a case, the second individual must share the blood type of the intended recipient or must have an immuno-neutral blood type, such as type O in humans. Alternatively, the red blood cell may have its immunological determinants masked by a substance such as PEG (see below).

As used herein, the term "red blood cell" refers to a living, enucleate red blood cell (i.e., a mature erythrocyte) of a vertebrate.

Preferably the red blood cell is a mammalian red blood cell, advantageously a. human red blood cell. As used herein, the term "mammal" refers to a member of the class Mammalia including, but not limited to, a rodent, lagomorph, pig or primate. Preferably, the mammal is a human.

As used herein the term "introducing" includes but is not limited to the administration of a red blood cell and/or an agent into a vertebrate.

As used herein in reference to administration of an agent to a vertebrate, the term "introducing" includes but is not limited to causing the agent to enter the circulatory system of the vertebrate by transfusion or to infusing an agent to a target site. It is contemplated that a hollow needle, such as a hypodermic needle or cannula, is inserted through the wall of a blood vessel (e.g., a vein or artery) and the red blood cell is either injected using applied pressure or allowed to diffuse or otherwise migrate into the blood vessel. It is understood that the diameter of the needle is sufficiently large and the pressure sufficiently light to avoid damage of the cell by shear forces. Preferably, introduction of a red blood cell into a vertebrate is intra-arterial or intravenous. Methods of blood cell transfusion are well known in the art.

### Immunocompatibility

The loaded red blood cell vehicles may be coated with an agent which masks cell surface antigens. For example, PEGylated red blood cells evade the host immune response and thereby enjoy prolonged circulation. According to one such method, methoxy (polyethylene glycol), or mPEG, is covalently bound to red blood cells (Scott *et al*., 1997, Proc. Natl. Acad. Sci. U.S.A., 94: 7566-7571). This procedure has been shown to result in a loss of ABO blood group reactivity and inhibition of phagocytic destruction by monocytes; in addition, the survival of mPEG treated sheep red blood cells transfused into mice is increased 360-fold over that of untreated control cells (Scott *et al.,* 1997, supra).

A second coating which may be useful is one which comprises distearoyl-phosphatidylethanolamine (DSPE)-conjugated PEG *(Du et al.,* 1997, Biochim. Biophys. Acta = Biomembranes, 1326: 236-248). When applied as a monolayer film to a glass plate, DSPE-PEG inhibits protein adsorption and cell adhesion to the glass plate (Du *et al*., 1997, supra).

### Targeting

According to a method disclosed in U.S. Patent No. 4,669,481, limited targeting of red blood cells to a small subset of vertebrate tissues is achieved, if desired, as follows: Treating the red blood cell under mild heating conditions will damage the cells, resulting in rapid sequestration by the reticuloendothelial system. The cells can be specifically targeted for the spleen by heating for 10 minutes at 49°C. Greater temperature or length of heating produces increased cell damage, with resultant hepatic uptake. Thus, if desired, payload delivery to the spleen or liver can be preferentially enhanced; however, the degree to which the payload is lost from damaged cells prior to administration is not known.

As used herein, the term "target" is used in reference to the spatial coordinates (anatomical location) of the cell, tissue or site (such as a vessel) to which the agent is delivered.

The red blood cells may be targeted to any desired site in a vertebrate, or mammal. As used herein, the term "site" refers to a region of the body of a vertebrate, which region may comprise an anatomical area, a tissue, a group of tissues, a cell, a group of cells or even substantially all of the cells of the vertebrate.

Preferably the target is a cell.

As used herein, the term "cell" refers to a viable, naturally-occurring or genetically engineered, single unit of an organism.

Preferably the target is a tissue.

As used herein, the term "tissue" refers to a population or physical aggregation of cells within an organism, wherein the cells are of the same cell type or are of cell different types resident within a single organ or other functional unit. As used herein, the term "tissue" refers to intact tissue or tissue fragments, such that the cells are sufficiently aggregated (associated) so as to form a cohesive mass. Alternatively, the term "tissue" refers to a collection of individual cells, such as those which circulate (e.g., in blood or lymphatic fluid) within the vertebrate. A tissue may comprise an entire organ (e.g. the pancreas, the thyroid, a muscle, bone or others) or other system (e.g. the lymphatic system) or a subset of the cells thereof; therefore, a tissue may comprise 0.1-10%, 20-50% or 50-100% of the organ or system (e.g., as is true of islets of the pancreas).

Preferably the target is a vessel.

As used herein the term "vessel" means any artery, vein or other "lumen" in an organism to which ultrasound can be applied and to and an agent may be delivered. A lumen is a channel within a tube or tubular organ. Examples of preferred vessels include but are not limited to the coronary artery, carotid artery, the femoral artery, and the iliac artery.

In one embodiment, an ultrasound energy source may be focused at the target cell, tissue or site (such as a vessel) as loaded red blood cells circulate through it. For example, a diagnostic and/or therapeutic ultrasound energy source or a combination thereof may be applied to a target tissue. This is particularly applicable to target tissues located on the surface of the subject vertebrate, although deep targets may also be treated with an ultrasound energy source.

### Agent

As used herein, the term "agent" includes but is not limited to an atom or molecule, wherein a molecule may be inorganic or organic. a biological effector molecule and/or a nucleic acid encoding an agent such as a biological effector molecule, a protein, a polypeptide, a peptide, a nucleic acid, a virus, a virus-like particle, a nucleotide, a ribonucleotide, a synthetic analogue of a nucleotide, a synthetic analogue of a ribonucleotide, a modified nucleotide, a modified ribonucleotide, an amino acid, an amino acid analogue, a modified amino acid, a modified amino acid analogue, a steroid, a proteoglycan, a lipid and a carbohydrate. An agent may be in solution or in suspension (e.g., in crystalline, colloidal or other particulate form). The agent may be in the form of a monomer, dimer, oligomer, etc, or otherwise in a complex.

The agent may be an imaging agent, by which term is meant an agent which may be detected. whether in *vitro* in the context of a tissue, organ or organism in which the agent is located. The imaging agent may emit a detectable signal, such as light or other electromagnetic radiation. The imaging agent may be a radio-isotope as known in the art, for example ³²P or ³⁵S or ⁹⁹Tc, or a molecule such as a nucleic acid, polypeptide, or other molecule as explained below conjugated with such a radio-isotope. The imaging agent may be opaque to radiation, such as X-ray radiation. The imaging agent may also comprise a targeting means by which it is directed to a particular cell, tissue, organ or other compartment within the body of an animal. For example, the agent may comprise a radiolabelled antibody specific for defined molecules, tissues or cells in an organism.

The imaging agent may be combined with, conjugated to, mixed with or combined with. any of the agents disclosed herein.

It will be appreciated that it is not necessary for a single agent to be used, and that it is possible to load two or more agents for into the vehicle. Accordingly, the term "agent" also includes mixtures, fusions, combinations and conjugates, of atoms, molecules etc as disclosed herein. For example, an agent may include but is not limited to: a nucleic acid combined with a polypeptide; two or more polypeptides conjugated to each other; a protein conjugated to a biologically active molecule (which may be a small molecule such as a prodrug): or a combination of a biologically active molecule with an imaging agent.

As used herein, the term "biological effector molecule" or "biologically active molecule" refers to an agent that has activity in a biological system, including, but not limited to, a protein, polypeptide or peptide including, but not limited to, a structural protein, an enzyme, a cytokine (such as an interferon and/or an interleukin) an antibiotic, a polyclonal or monoclonal antibody, or an effective part thereof, such as an Fv fragment, which antibody or part thereof may be natural, synthetic or humanised, a peptide hormone, a receptor, a signalling molecule or other protein; a nucleic acid, as defined below, including, but not limited to, an oligonucleotide or modified oligonucleotide, an antisense oligonucleotide or modified antisense oligonucleotide, cDNA, genomic DNA, an artificial or natural chromosome (e.g. a yeast artificial chromosome) or a part thereof, RNA, including mRNA, tRNA, rRNA or a ribozyme, or a peptide nucleic acid (PNA); a virus or virus-like particles; a nucleotide or ribonucleotide or synthetic analogue thereof, which may be modified or unmodified; an amino acid or analogue thereof, which may be modified or unmodified; a non-peptide (e.g., steroid) hormone; a proteoglycan; a lipid; or a carbohydrate. If the biological effector molecule is a polypeptide, it may be loaded directly into a red blood cell as described; alternatively, a nucleic acid molecule bearing a sequence encoding the polypeptide, which sequence is operatively linked to transcriptional and translational regulatory elements active in a cell at the target site, may be loaded. Small molecules, including inorganic and organic chemicals, are also of use in the methods described here. In a particularly preferred embodiment, the biologically active molecule is a pharmaceutically active agent, for example, an isotope.

Particularly useful classes of biological effector molecules include, but are not limited to, antibiotics, anti-inflammatory drugs, angiogenic or vasoactive agents, growth factors and cytotoxic agents (e.g., tumour suppressers). Cytotoxic agents include, but are not limited to, diptheria toxin, Pseudomonas exotoxin, cholera toxin, pertussis toxin, and the prodrugs peptidyl-p-phenylenediamine-mustard, benzoic acid mustard glutamates. ganciclovir, 6-methoxypurine arabinonucleoside (araM), 5-fluorocytosine, glucose, hypoxanthine, methotrexate-alanine, N-[4-(a-D-galactopyranosyl) benyloxycarbonyl]-daunorubicin, amygdalin, azobenzene mustards, glutamyl p-phenylenediamine mustard, phenolmustard-glucuronide, epirubicin-glucuronide, vincacephalosporin,phenylenediamine mustard-cephalosporin, nitrogen-mustard-cephalosporin, phenolmustard phosphate, doxorubicin phosphate, mitomycin phosphate, etoposide phosphate, palytoxin-4-hydroxyphenyl-acetamide, doxorubicin-phenoxyacetamide, melphalan-phenoxyacetamide, cyclophosphamide, ifosfamide or analogues thereof. If a prodrug is loaded in inactive form, a second biological effector molecule may be loaded into the red blood cell. Such a second biological effector molecule is usefully an activating polypeptide which converts the inactive prodrug to active drug form, and which activating polypeptide is selected from the group that includes, but is not limited to, viral thymidine kinase (encoded by Genbank Accession No. J02224); carboxypeptidase A (encoded by Genbank Accession No. M27717), α-galactosidase (encoded by Genbank Accession No. M13571), β-glucuronidase (encoded by Genbank Accession No. M15182), alkaline phosphatase (encoded by Genbank Accession No. J03252 J03512), or cytochrome P-450 (encoded by Genbank Accession No. D00003 N00003), plasmin. carboxypeptidase G2, cytosine deaminase, glucose oxidase, xanthine oxidase, β-glucosidase, azoreductase, t-gutamyl transferase, β-lactamase, or penicillin amidase. Preferably, the polypeptide capable of activating a prodrug is DT diaphorase. Either the polypeptide or the gene encoding it may be loaded; if the latter, both the prodrug and the activating polypeptide may be encoded by genes on the same recombinant nucleic acid construct. Preferably the biological effector molecule is selected from the group consisting of a protein, a polypeptide, a peptide, a nucleic acid, a virus, a virus-like particle, a nucleotide, a ribonucleotide, a synthetic analogue of a nucleotide, a synthetic analogue of a ribonucleotide, a modified nucleotide, a modified ribonucleotide, an amino acid, an amino acid analogue, a modified amino acid, a modified amino acid analogue, a steroid, a proteoglycan, a lipid and a carbohydrate or a combination thereof (e.g., chromosomal material comprising both protein and DNA components or a pair or set of effectors, wherein one or more convert another to active form, for example catalytically).

The methods advantageously employ agents which are not able to diffuse through an intact erythrocyte cell wall by passive or active means. However, the delivery of agents which diffuse at a certain rate through the erythrocyte cell wall is contemplated, particularly where increased delivery of the agent at a particular time or location is desirable. Increased delivery may be achieved by ultrasound administration at the appropriate time or location.

The agents, including biological effector molecules, may also be delivered into cells as fusions (for example, protein or polypeptide fusions) or conjugates with a protein capable of crossing the plasma membrane and/or the nuclear membrane. Preferably, the agent/biological effector molecule is fused or conjugated to a domain or sequence from such a protein responsible for the translocational activity. Preferred translocation domains and sequences include domains and sequences from the HIV-1-trans-activating protein (Tat), *Drosophila* Antennapedia homeodomain protein and the herpes simpiex-1 virus VP22 protein. By this means, the agent/biological effector molecule is able to enter the cell or its nucleus when released in the vicinity of the cell using the methods described herein.

Exogenously added HIV-1-trans-activating protein (Tat) can translocate through the plasma membrane and to reach the nucleus to transactivate the viral genome. Translocational activity has been identified in amino acids 37-72 (Fawell *et al*., 1994, *Proc. Natl. Acad. Sci. U.S.A.* 91, 664-668), 37-62 (Anderson *et al*., 1993, *Biochem. Biophys. Res. Commun.* 194, 876-884) and 49-58 (having the basic sequence RKKRRQRRR) of HIV-Tat. Vives et al. (1997), *J Biol Chem* 272, 16010-7 identified a sequence consisting of amino acids 48-60 (CGRKKRRQRRRPPQC), which appears to be important for translocation, nuclear localisation and trans-activation of cellular genes. Intraperitoneal injection of a fusion protein consisting of β-galactosidase and a HIV-TAT protein transduction domain results in delivery of the biologically active fusion protein to all tissues in mice *(Schwarze* et al., 1999, Science 285, 1569-72)

The third helix of the *Drosophila* Antennapedia homeodomain protein has also been shown to possess similar properties (reviewed in Prochiantz, A., 1999, *Ann N Y Acad Sci,* 886, 172-9). The domain responsible for translocation in Antennapedia has been localised to a 16 amino acid long peptide rich in basic amino acids having the sequence RQIKIWFQNRRMKWKK (Derossi, et al., 1994, *J Biol Chem,* 269, 10444-50). This peptide has been used to direct biologically active substances to the cytoplasm and nucleus of cells in culture (Theodore, et al., 1995, *J. Neurosci* 15, 7158-7167). Cell internalization of the third helix of the Antennapedia homeodomain appears to be receptor-independent, and it has been suggested that the translocation process involves direct interactions with membrane phospholipids (Derossi et al., 1996, J Biol Chem, 271, 18188-93). The VP22 tegument protein of herpes simplex virus is capable of intercellular transport, in which VP22 protein expressed in a subpopulation of cells spreads to other cells in the population (Elliot and O'Hare, 1997, *Cell* 88, 223-33). Fusion proteins consisting of GFP (Elliott and O'Hare, 1999, *Gene Ther* 6, 149-51), thymidine kinase protein (Dilber et al., 1999, *Gene Ther* 6, 12-21) or p53 (Phelan et al., 1998, *Nat Biotechnol* 16, 440-3) with VP22 have been targeted to cells in this manner.

Particular domains or sequences from proteins capable of translocation through the nuclear and/or plasma membranes may be identified by mutagenesis or deletion studies. Alternatively, synthetic or expressed peptides having candidate sequences may be linked to reporters and translocation assayed. For example, synthetic peptides may be conjugated to fluoroscein and translocation monitored by fluorescence microscopy by methods described in Vives *et al.,* (1997), *J Biol Chem* 272, 16010-7. Alternatively, green fluorescent protein may be used as a reporter (Phelan *et al.,* 1998, *Nat Biotechnol* 16, 440-3).

Any of the domains or sequences or as set out above or identified as having translocational activity may be used to direct the agents (including biological effector molecules) into the cytoplasm or nucleus of a cell.

### Nucleic Acid

A nucleic acid of use in the methods described may comprise a viral or non-viral DNA or RNA vector, where non-viral vectors include, but are not limited to, plasmids. linear nucleic acid molecules, artificial chromosomes and episomal vectors. Expression of heterologous genes has been observed after injection of plasmid DNA into muscle (Wolff J. *A. et al.,* 1990, Science, 247: 1465-1468; Carson D.A. *et al.,* US Patent No. 5,580,859), thyroid (Sykes *et al.,* 1994, Human Gene Ther., 5: 837-844), melanoma (Vile *et al.,* 1993, Cancer Res., 53: 962-967), skin (Hengge *et al.,* 1995, Nature Genet., 10: 161-166), liver (Hickman *et al.,* 1994, Human Gene Therapy, 5: 1477-1483) and after exposure of airway epithelium (Meyer *et al.,* 1995, Gene Therapy, 2: 450-460).

As used herein, the term "nucleic acid" is defined to encompass DNA and RNA or both synthetic and natural origin which DNA or RNA may contain modified or unmodified deoxy- or dideoxy- nucleotides or ribonucleotides or analogues thereof. The nucleic acid may exist as single- or double-stranded DNA or RNA, an RNA/DNA heteroduplex or an RNA/DNA copolymer, wherein the term "copolymer" refers to a single nucleic acid strand that comprises both ribonucleotides and deoxyribonucleotides. The term "nucleic acid" is also intended to include oligonucleotides and modified oligonucleotides.

The term "synthetic", as used herein, is defined as that which is produced by *in vitro* chemical or enzymatic synthesis.

Therapeutic nucleic acid sequences useful according to the methods described here include those encoding receptors, enzymes, ligands, regulatory factors, and structural proteins. Therapeutic nucleic acid sequences also include sequences encoding nuclear proteins, cytoplasmic proteins, mitochondrial proteins, secreted proteins, plasmalemma-associated proteins, serum proteins, viral antigens, bacterial antigens, protozoal antigens and parasitic antigens. Useful therapeutic nucleic acid sequences also include sequences encoding proteins, lipoproteins, glycoproteins, phosphoproteins and nucleic acids (e.g., RNAs such as ribozymes or antisense nucleic acids). Ribozymes of the hammerhead class are the smallest known, and lend themselves both to *in vitro* synthesis and delivery to cells (summarised by Sullivan, 1994, J. Invest. Dermatol., 103: 85S-98S; Usman *et al.,* 1996, Curr. Opin. Struct. Biol., 6: 527-533). Proteins or polypeptides which can be expressed by nucleic acid molecules delivered according to the present invention include hormones, growth factors, neurotransmitters, enzymes, clotting factors, apolipoproteins, receptors, drugs, oncogenes, tumour antigens, tumour suppressers, structural proteins, viral antigens, parasitic antigens and bacterial antigens. The compounds which can be incorporated are only limited by the availability of the nucleic acid sequence encoding a given protein or polypeptide. One skilled in the art will readily recognise that as more proteins and polypeptides become identified, their corresponding genes can be cloned into the gene expression vector(s) of choice, administered to a tissue of a recipient patient or other vertebrate, and expressed in that tissue.

### Delivery of Agents

The methods described here are useful for the delivery of agents to a selected site in a vertebrate body, whether an organ, part of an organ or otherwise, in the presence or absence of specific targeting means. This is achieved, as set out above, by the selective disruption by ultrasound at the selected target site of electrosensitised red blood cells loaded with the agent of choice.

Preferred agents include those useful for imaging of tissues in *vivo* or *ex vivo*. For example, imaging agents, such as labelled antibodies which are specific for defined molecules. tissues or cells in an organism, may be used to image specific parts of the body by releasing them at a desired location using ultrasound. This allows imaging agents which are not completely specific for the desired target, and which might otherwise lead to more general imaging throughout the organism, to be used to image defined tissues or structures. For example, an antibody which is capable of imaging endothelial tissue may be used to image liver vasculature by releasing the antibody selectively in the liver by applying ultrasound thereto.

### Kits

We describe a kit comprising a red blood cell, an agent and packaging materials therefor.

A kit may be designed for the easy delivery of an agent to a recipient vertebrate, whether in a research or clinical setting. A kit takes one of several forms, as follows:

A kit for the delivery of an agent to a subject vertebrate comprises red blood cells and the agent and instructions for performing the methods described here. Alternatively, the red blood cells are supplied loaded with the agent for convenience of use by the purchaser. The cells are supplied sensitised for rapid use or, for greater stability, unsensitised. In the latter case, the sensitising process is carried out separately from the cells. The cells of the kit are species-specific to the vertebrate of interest, such as a primate, including a human, canine, rodent, pig or other, as desired; in other words, the cells are of like species with the intended recipient. The cells of the kit are, additionally, specific to the blood type of the intended recipient organism, as needed. Optionally, the kit comprises one or more buffers for cell sensitisation, washing, resuspension, dilution and/or administration to a vertebrate. Appropriate buffers are selected from the group that includes low ionic strength saline, physiological buffers such as PBS or Ringer's solution, cell culture medium and blood plasma or lymphatic fluid. The kit additionally comprises packaging materials (such as tubes, vials, bottles, or sealed bags or pouches) for each individual component and an outer packaging, such as a box, canister or cooler, which contains all of the components of the kit. The kit is shipped refrigerated. Optionally, non-cellular components are supplied at room temperature or frozen, as needed to maintain their activity during storage and snipping. They may be in liquid or dry (i.e., powder) form.

A second kit comprises an agent such as a biological effector molecule, instructions for performing the methods described here and, optionally a sensitising device and buffers therefor (e.g., saline or other physiological salt buffer, culture medium, plasma or lymphatic fluid). In addition, the kit contains appropriate packaging materials, as described above. The individual components may be supplied in liquid or dry (i.e., powder) form, and may be at room temperature, refrigerated or frozen as needed to maintain their activity during storage and shipping. Red blood cells for use with this kit are obtained independently (for example, they may be harvested from the intended recipient vertebrate).

The kit may comprise a red blood cell which is loaded with an agent, and packaging materials therefor.

Preferably, a kit as described above further comprises an apparatus for applying the sensitising procedure.

Preferably the kit further comprises polyethylene glycol.

It is additionally preferred that the kit further comprises a liquid selected from the group consisting of a buffer, diluent or other excipient.

Preferably, the liquid is selected from the group consisting of a saline buffer, a physiological buffer and plasma. We further describe a physiological composition comprising a red blood cell comprising a biological effector molecule admixed with a physiologically compatible buffer. As used herein, the term "physiologically compatible buffer" or "physiological buffer" is defined as a liquid composition which, when placed in contact with living cells, permits the cells to remain alive over a period of minutes, hours or days. As such, a physiological buffer is substantially isotonic with the cell, such that cell volume does not change more than 20% due to differences in internal and external ionic strength. Non-limiting examples of physiologically compatible buffers or physiological buffers include dilute saline, which may be buffered (e.g., Hanks' buffered saline or phosphate buffered saline), or other physiological salts (e.g., Ringer's solution), dilute glucose, sucrose or other sugar, dilute glycerol with- or without salts or sugars, cell culture media as are known in the art, serum and plasma.

Preferably, the red blood cell of the physiological composition is human.

### EXAMPLES

### Example 1

### Electric field-mediated sensitivity of human red blood cells and polyethylene glycol (PEG)-treated red blood cells to ultrasound in PBS/Mg/glucose buffer

The responses of normal cells were compared with those of electroporated and re-sealed cells following exposure to ultrasound over a range of power densities. The responses of normal cells were also compared with those of polyethylene glycol (PEG) treated cells which had been electroporated and re-sealed under similar conditions. To this end human blood was harvested by venipuncture and washed twice in PBS (phosphate buffered saline) by centrifugation. Cells were suspended in PBS containing 1mg/ml fluorescein to yield concentrations of 7×10⁸ cells/ml and 0.8ml aliquots were dispensed into electroporation cuvettes (0.4cm electrode gap) and retained on ice for 10min. Cells were then exposed to an electroporation strategy involving delivery of two electric pulses (field strength = 3.625kV/cm at a capacitance of 1 µF) using a BioRad Gene Pulser apparatus. Cells were immediately washed with PBS containing MgCl₂ (4mM) (PBS/Mg) and retained at room temperature for 30min. in the PBS/Mg (containing 1mg/ml fluorescein) buffer to facilitate re-sealing. Aliquots of those cells were treated with polyethylene glycol (av. mol. Weight = 5000) as described by Scott *et al.*, 1997 Proc. Natl. Acad. Sci. (U.S.A.), 94, 7566-7571 using cyanuric chloride treated methoxy polyethylene glycol at a concentration of 25mg/ml. Cells were subsequently washed and suspended at a concentration of 14x10⁸ cells/ml in PBS/Mg containing 10mM glucose (PBS/Mg/glucose) for at least 1 hour. Samples were treated with ultrasound by dispensing 0.1 ml aliquots of cells into microwells (inner diameter = 5mm). Ultrasound power densities were generated using a Rich-Mar Multi Hz generator fitted with a 3MHz ultrasound head (U.S.A.) and set to delivery continuous wave ultrasound at the required power density. Samples were treated for 30seconds and cell counts were subsequently determined using a haemocytometer. In addition, samples of cells were analysed using a Becton Dickinson flow cytometer in order to determine leakage of fluorescein following exposure to ultrasound.

### Results 1

The results are shown in Fig.1 and they demonstrate that the ultrasound power densities between 0.5 and 1.5W/cm² had little or not effect on either normal or PEG-treated red blood cells. However, when electroporated normal and PEG-treated cells were exposed to increasing ultrasound power densities very significant lysis was detected, particularly at densities above 1 W/cm². The results demonstrate that cells which had been treated using conditions suitable for electroporative loading of materials into human red blood cells were rendered hyper-sensitive to ultrasound during that loading procedure. In addition to examining cell lysis it was also decided to employ flow cytometry to determine whether or not the pay-load was released during exposure to ultrasound. Again the results are shown in Fig. 1 and it was found that when PEG-treated cells loaded with fluorescein were exposed to increasing ultrasound power densities the geometric mean of fluorescence decreased. The control population for this experiment consisted of PEG-treated cells which were exposed to fluorescein in the absence of exposure to electric field conditions. The results demonstrate ultrasound-mediated leakage of a pay-load from the hyper-sensitised, PEG-treated red blood cells.

### Example 2

### Electric field-mediated sensitivity of human red blood cell and polyethylene glycol-treated red blood cells to ultrasound in autologous plasma

The data in Example 1 demonstrated that cells which had been treated with electric field conditions suitable for loading human red blood cells were rendered hyper-sensitive to ultrasound. This degree of sensitivity remained following storage of the samples for 1 hour in PBS/Mg/glucose buffer. It was of interest to determine whether or not the electro-sensitised cells exhibited sensitivity to ultrasound in the presence of autologous plasma. To this end both normal and PEG-treated human red blood cells were electro-sensitised in the presence of fluorescein as described for Example 1. Cells were allowed to re-seal in PBS/Mg (containing fluorescein) for 30min. and subsequently placed in PBS/Mg/glucose for 15 min. Cells were then suspended in autologous plasma at a concentration of approximately 14x10⁸ cell/ml. Cells were stored at room temperature for 1 hour and then treated with ultrasound at the indicated power densities and cell counts determined as described for Example 1. In addition, samples of cells were analysed using a Becton Dickinson flow cytometer in order to determine leakage of fluorescein following exposure to ultrasound.

### Results 2

The results are shown in Fig. 2A and they demonstrate that exposing normal and PEG-treated control cell populations in the presence of plasma to ultrasound has little or not effect on those cells at power densities ranging from 0.25 - 1.5W/cm². However, exposure of the electro-sensitised normal and PEG-treated cells to ultrasound in the presence of plasma results in increasing cell lysis with increasing power density (Fig 2A). These results demonstrate that the susceptibility of both electro-sensitised human red blood cells and PEG-treated human red blood cells to ultrasound remains in the presence of autologous plasma. In addition, it was found that ultrasound-mediated release of the loaded fluorescein was achieved following exposure of the electro-sensitised, PEG-treated cells to increasing power densities as demonstrated by a decrease in the geometric mean of fluorescence using flow cytometry (Fig.2B). Control populations of cells consisted of exposing PEG-treated cells exposed to fluorescein in the absence of an electo-sensitisation event (Fig 2B). These results demonstrate ultrasound-mediated leakage of a pay-load from the hyper-sensitised, PEG-treated red blood cells in the presence of autologous plasma.

### Example 3

### Stability of electric field-mediated sensitisation of normal and PEG-treated human red blood cells to ultrasound during prolonged storage in PBS/Mg/Glucose

Examples 1 and 2 demonstrated that exposure of both normal and PEG-treated red blood cells to electric field conditions suitable for loading cells coincidentally conferred upon those cells hyper-sensitivity to ultrasound. It was decided to determine whether or not hyper-sensitivity persisted during storage. Cells were harvested and electro-sensitised as described for Example 1. Cells were subsequently re-sealed in PBS/Mg for 30 min. and a proportion were treated with PEG as described by Scott et *al*. (1997) Proc. Natl. Acad. Sci. (USA), 94, 7566-7571). Cells were then suspended in PBS/Mg/glucose (supplemented with 1% v/v penicillin/streptomycin solution [5000IU/ml] and 1% v/v gentamicin [10mg/ml]) to yield concentrations of 14x10⁸ cells/ml. Cells were stored at 4°C and samples were treated with ultrasound (1.25W/cm² for 30 seconds using a 3MHz ultrasound head) as described in Example 1. The degree of cell lysis was determined using a hemocytometer.

### Results 3

The results are shown in Fig.3 and they demonstrate that both normal and PEG-treated cells exhibit little or no susceptibility to ultrasound during storage over a 7 day period. The results also demonstrate that the degree of ultrasound sensitivity induced by electro-sensitisation in both red blood cells and PEG-treated red blood cells is retained over this period of time. It was concluded from this experiment that the electro-sensitisation phenomenon exhibited by both the red blood cells and the PEG-treated red blood cells is retained during storage at 4°C in PBS/Mg/glucose.

### Example 4

### Stability of electric field-mediated sensitisation of normal and PEG-treated human red blood cells to ultrasound during storage in autologous plasma

It has been shown in Example 3 that cells which had been rendered ultrasound sensitive using electro-sensitisation remained sensitive for prolonged periods of time during storage on PBS/Mg/glucose. It was of interest to determine whether or not this phenomenon could be retained for prolonged periods of time during storage in autologous plasma. To this end both sensitised and non-sensitised normal and PEG-treated cells were prepared as described for Example 3. Samples were re-sealed for 30min in PBS/Mg and subsequently transferred to PBS/Mg/glucose for 15min. Cells were then suspended in autologous plasma supplemented with 1% v/v penicillin/streptomycin solution [5000IU/ml] and 1% v/v gentamicin [10mg/ml]) to yield concentrations of 13x10⁸ cells/ml. Cells were stored at 4°C and samples were treated with ultrasound as described in Example 3.

### Results 4

The results obtained are shown in Fig.4 and they demonstrate that normal and PEG-treated red blood cells stored in autologous plasma for 6 days remained insensitive to ultrasound. However, electro-sensitised normal and PEG-treated cells exhibited hyper-sensitivity to ultrasound over the time period examined. These results demonstrate that the electro-sensitised cells retained their sensitivity even over prolonged periods of time in autologous plasma.

### Example 5

### The effect of electroporation conditions on sensitivity of human red blood cells to ultrasound

Since the above results demonstrated that exposure of human red blood cells to short duration (0.02 mseconds) double electric pulses of 1.45kV (3.625kV/cm) at a capacitance of 1 uF conferred upon those cells sensitivity to lysis by ultrasound it was decided to determine whether or not alternative electric pulse conditions might yield similarly sensitised cells. The wave form of the above pulses is referred to as and exponential and describes delay of the delivered pulse across the electrodes. Alternatives include either a square wave or a modulated wave decay across the electrodes in the electroporation cuvettes. In order to examine a representative range of electrical conditions it was decided to study a number of parameters associated with exponential wave decay. These included pulse number delivered to the cuvettes, the capacitance of the pulse(s) delivered, the voltage of each pulse and the effect of delivering sequential pulses at varying voltages. Cells were suspended at not greater than 7×10⁸ cells/ml in PBS, exposed to the conditions described in Table 1 and finally suspended in PBS/Mg/glucose for at least 1 hour prior to exposure to ultrasound. Conditions for exposure to ultrasound were those described for Example 1. Cell lysis was determined by counting surviving cells. In addition, it was decided to examine the effects of delivering both square wave and modulated wave pulses to cells and to assess such delivery in terms of susceptibility to ultrasound. In these cases cells were exposed to electric pulses in electroporation cuvettes with a 0.2cm electrode gap. Cells were again suspended in PBS during delivery of pulses and subsequently exposed to ultrasound following suspension in PBS/Mg/glucose for 1hour. Cell lysis was determined by counting cells remaining after treatment with ultrasound. In all of the above studies control populations of cells, which had not been exposed to electric pulses, were treated with ultrasound and cell lysis was determined by cell counting.

### Results 5

The results obtained in this series of studies are shown in Table 1 below and they and a number of features in terms of inducing ultrasound sensitivity are evident: (i) Ultrasound sensitivity may be induced using pulses delivered as exponential, square or modulated wave forms. (ii) Increasing the pulse number using exponential wave delivery increases red blood cell sensitivity to ultrasound at lower voltage (see Table 1 ; 0.7kV, 1uF single and double exponential pulse). (iii) Increasing the capacitance at lower pulse voltages increases the ultrasound sensitivity (Table 1; 0.6kV, 1uF). (iv) Sequential delivery of low, high and finally low voltage using exponential wave form results in ultrasound sensitivity. (v) Ultrasound sensitivity may be induced using pulses with exponential, square and modulated wave forms. These results demonstrate that ultrasound sensitivity may be induced using a relatively wide variety of electrical parameters.

**Table 1 ELECTROPORATION CONDITIONS SUITABLE FOR SENSITISATION TO ULTRASOUND**

| **Conditions** | **U/S sensitivity % cell lysis** |
|---|---|
| **Exponential wave**^{**+**} | |
| **Single pulse** | |
| 0.7kV, 1 uF | 15 |
| 1kV, 1uF | 80 |
| 1.45kV, 1uF | 100 |
| **Double pulse** | |
| 0.7kV, 1uF | 84 |
| 1kV, 1uF | 88 |
| 1.45kV, uF | 96 |

| **Sequential pulsing** | |
|---|---|
| 0.3kV, 10uF: 1.45kV, 1uF: 0.3kV, 10uF | 98 |
| **Increased capacitance** | |
| 0.6kV, 1 uF | 4 |
| 0.6kV, 10uF | 86 |

| **Using BioRad RF module* (square wave)** | |
|---|---|
| 0.1 kV, 40kHz, 100ms burst, 1s burst interval, 5 bursts | |
| 0% modulation (square wave) | 90 |
| 100% modulation | 91 |

| | |
|---|---|
| *+ All samples were treated in 0.4cm cuvettes and electric pulse conditions were delivered using a BioRad Gene Pulsar apparatus*. *Voltages refer to those delivered by the apparatus*. | |
| ** The BioRad RF module is designed to deliver either square wave or modulated wave pulses. In all of the above studies pulses were delivered to cells suspended in PBS*. *Cells were exposed to ultrasound as described for Example I using 1.25W*/*cm*^{*2*} *for 30s. In all cases control populations of cells at the same concentration were exposed to ultrasound In those control sample no significant lysis was detected following exposure to ultrasound* | |

### Example 6

### Effect of cell concentration on electric field mediated sensitisation of human red blood cells to ULTRASOUND.

The purpose of this study was to determine whether increasing the concentration of the electro-sensitised cells resulted in decreased responses to ultrasound. In order to determine whether or not this might be the case samples of cells were harvested as described for Example 1 and cell concentrations were adjusted to 8 and 14x10⁸ cells/ml. Populations were then electroporated, placed in PBS/Mg for 30 minutes to re-seal and subsequently suspended in PBS/Mg/glucose for storage at room temperature. Both populations were stored for 1h and 3 h prior to treatment with ultrasound as described for Examples above (3MHz, 1.5W/cm², 5min.) and cell counts were determined 30min. after treatment. Control samples were treated in a similar manner although the electroporation event was omitted.

### Results 6

The results are shown in Fig. 5 and they indicate little or no effect in control samples (C8 and C 14) for both cell concentrations. When cells were stored for 1h and treated with ultrasound, lysis in both populations was 98-99% (Fig. 5; Samples 1 and 2). However when samples were stored for 3h and subsequently treated with ultrasound, lysis of the population containing 8x10⁸ cells/ml was 99% whereas that of the population containing 14x10⁸ cells/ml was only 15% (Fig.5; Samples 3 and 4, respectively). These results suggested that cells at the higher concentration had the ability to recover from electro-sensitisation during storage. Since a higher concentration of cells had been employed in the electro-sensitisation process it seemed reasonable to presume that electric field-mediated effects on individual cells within the overall population would be reduced. This may have resulted in the ability of those individual cells to recover from sensitisation. In order to test this hypothesis, cells were harvested and electroporated in aliquots containing 8x10⁸ cells/ml. Cells were allowed to recover in PBS/Mg at that concentration for 30min. and subsequently pooled to yield 14x10⁸ cells/ml in PBS/Mg/glucose for storage (RT). Cells were treated with ultrasound following 3h storage and cell counts were determined 30min. later. Control samples (C14*) were treated in a similar manner without delivery of the electro-sensitisation pulses. The results are shown in Fig.5 (Sample 5) and it would found that 99% of the cells lysed following treatment. These results confirmed out hypothesis and demonstrated that in order for ultrasound-sensitivity to persist at higher cell concentrations it would be necessary to perform the electro-sensitisation procedure at lower cell concentrations. It would alternatively suggest that electro-sensitisation of human red blood cells at higher cell concentrations would require adjustment of the electrical parameters delivered to those cells in order to sustain ultrasound sensitivity during storage.

### Example 7

### Effects of ultrasound on electro-sensitised human red blood cells placed in a soft tissue phantom

The results above demonstrate that electro-sensitisation of human red blood cells to ultrasound may be achieved in such a manner that those cells may be selectively lysed using ultrasound parameters which have little or no effect on normal human red blood cells *in vitro.* The purpose of this study was to demonstrate the selective effect *in vivo*. To this end a soft tissue Doppler phantom was employed (supplied by Dansk Fantom Service, Denmark). The phantom consists of a matrix which transmits sound at a mean velocity of 1503ms⁻¹ at 3MHz, attenuates sound at 0.54dBcm⁻¹* MHz and has a density of 1040Kgm⁻³. The system contains tubing with an inner diameter of 1.6mm and an outer diameter of 3mm which travels through the matrix at a 25° angle to the scanning surface. Samples of cells (7-8x10⁸) were injected into the tubing such that each sample treated was at an average depth of 1 cm from the scanning surface. Cells used in the system were harvested, electro-sensitised as described above in Example 1, re-sealed in PBS/Mg for 30min, and subsequently injected into the phantom. Control samples were treated in a similar manner except that electro-sensitisation was not carried out. Samples were treated with the 3MHz and 1 MHz ultrasound heads for 5 min.

### Results 7

The results obtained are shown in Fig.6 and they demonstrate that at 3MHz treatment resulted in an average of 7% lysis whereas treatment of the electro-sensitised samples resulted in 24% lysis. At 1 MHz treatment resulted in 3% lysis in control samples and 34% lysis in samples which had been electro-sensitised. These results demonstrated that preferential lysis of electro-sensitised human red blood cells may be achieved in a soft tissue vascular mimicking system. The results suggest that ultrasound could be exploited as a non-invasive means of releasing pay-load from a delivery vehicle which has been electro-sensitised.

### Example 8

### Electrosensitisation

To demonstrate that electrosensitisation of red blood cells occurs in the absence of electroporation under consditions of insufficient electric field energy to achieve electroporation, an experiment was designed in which a FITC-labelled polyclonal antiserum was loaded into red blood cells by electroporation. Cell lysis in response to ultrasound was assessed on a haemocytometer as in Example 1. The field strength was then reduced was then reduced to a point at which no antibody loading was observed, and cell lysis in resoponse to ultrasound was again assessed.

In a first experiment, 4.5x10⁷ RBCs were incubated with 0.25 mg/ml antibody, cooled to 4°C and electrosensitised by pulsing at 0.3 KV 10 µF, 1.45 KV 1 µF and 0.3 KV 10 µF in phosphate-buffered sucrose (PBSucrose).

In a second experiment, 3.5x10⁷ RBCs were incubated with 0.25 mg/ml antibody, cooled to 4°C and electrosensitised by pulsing at 0.3 KV 10 µF, 1.45 KV 1 µF and 0.3 KV 10 µF in PBS.

In a third experiment, 5x10⁷ RBCs were incubated with 0.5 mg/ml antibody, cooled to 4°C and electrosensitised by pulsing twice at 1.45 KV 1 µF in PBS.

### Results 8

The first and second experiments, pulsing at 0.3 KV 10 µF, 1.45 KV 1 µF and 0.3 KV 10 µF in PBS or PBSucrose, showed antibody loading at ratios of 3.98 and 4.04 respectively as determined by FACS (Figure 7). The cells were also 100% sensitive to ultrasound as determined by haemocytometer counting, according to Example 1.

The third experiment, pulsing twice at 1.45 KV 1 µF in PBS, showed no antibody loading when analysed by FACS (Figure 7). However, the cells remained 100% sensitive to ultrasound as determined by haemocytometer counting.

### Example 9

### Electro-sensitisation of human red blood cells treated with a hypotonic dialysis loading protocol.

The objective of these experiment was to determine whether or not it would be possible to electro-sensitise human red blood cells which may be loaded using alternative loading modalities. It was decided to employ a protocol designed to achieve loading using hypotonic dialysis as described by Eichler *et al.,* 1986, Clin. Pharmacol. Therap. 40: 300-303. Essentially, washed red blood cells were suspended in 1ml of PBS (150mM NaCl, 5mM K₂HPO₄/KH₂PO₄; pH 7.4) to obtain a hematocrit of 60%. This suspension was placed in dialysis tubing (molecular weight cutoff 12-14,000; Spectra-Por) and swelling of cells was obtained by dialysis against 100ml of 5mM K₂HPO₄/KH₂PO₄, pH 7.4 for 90 minutes at 4°C. Resealing was achieved by subsequent dialysis for 15 minutes at 37°C against 100ml of PBS containing 10mM glucose. Cells were then washed using centrifugation. In cases where electro-sensitisation was performed the method described in Example 1 was employed and cells were subsequently placed in PBS/Mg (Example 1) for 30min at room temperature. All cells were subsequently stored in PBS/Mg/glucose (Example 1) prior to treatment with ultrasound.

### Results 9

Following hypotonic dialysis and exchange into PBS/Mg/glucose, cells were stored at room temperature for 1 hour. Both cells treated with hypotonic dialysis (HD) and electro-sensitised cells which had been treated with hypotonic dialysis (ESHD) were then exposed to ultrasound at power densities of 1.25, 1.5 and 1.75W/cm² and at a frequency of 3MHz as described for Example 1. No significant lysis was observed in electro-sensitised samples above that exhibited by control cells which were treated with hypotonic dialysis alone. Indeed at 1:25W/cm² no lysis was observed in either sample and this contrasts significantly with results presented in Fig.1, Example 1 where electro-sensitised normal cells exhibited almost 100% lysis at that power density. When both the HD and ESHD cells were stored overnight at 4°C and subsequently exposed to ultrasound, no significant lysis was detected. It was subsequently decided to electro-sensitise the HD cells which had rested overnight. Following electro-sensitisation, cells were placed in PBS/Mg (Example 1) for 30 min. and subsequently placed in PBS/Mg/glucose for 1 hour. Cell (at a concentration of 5x10⁸ cells/ml were exposed to ultrasound at 1.25, 1.5 and 1.75 W/cm² as described above and the degree of lysis was determined. The results are shown in Fig. 8 and they demonstrate that the electro-sensitised samples exhibited preferential lysis following exposure to ultrasound at 1.5 and 1.75W/cm². The results from these experiments demonstrate that it is possible to render human red blood cells sensitive to ultrasound following treatment with alternative procedures designed to achieve loading of those cells. However, cell treated with the hypotonic dialysis method must be allowed to rest for a period of time prior to electro-sensitisation.

### EXAMPLE 10

### Release of payload from loaded and sensitised vehicle in a tissue mimicking system (TMM).

Since the previous examples demonstrated that human erythrocytes could be sensitised to low intensity ultrasound, it was decided to show that a payload entity loaded into those sensitised erythrocytes could be released following exposure to low intensity ultrasound. In these experiments the target is placed at a distance of 1.3cm from the emitting surface of the ultrasound head and the intervening space is filled with a tissue mimicking material (TMM) which attenuates ultrasound in the same manner as a soft tissue. The TMM chosen for this work is described in Madsen *et al.* (1998, Ultrasound Med. & Biol., 24, 535-542) and following preparation, care is taken to ensure that the material has a density of 1.03 g/ml.

In previous examples sensitised cells were employed as the target. In Example 9 above it is shown that cells which have been processed using technologies designed to load erythrocytes can be rendered sensitive by exposure to electric pulses. In order to demonstrate ultrasound-mediated payload release from the vehicle it was decided to employ a loaded vehicle as the target.

It was decided to employ a loading modality which incorporated a pre-sensitising step prior to loading by hypotonic dialysis and subsequent exposure of those cells to an electrosensitising step. The pre-sensitising step is an optional step which increases the efficiency of a subsequent loading step, and is described in detail in our co-pending British Patent Application GB0002856.3. Thus, in this and the following examples in which RBCs are loaded with payload, a pre-sensitising step is conducted to ensure optimal loading of payload into the RBCs. After loading of the agent, the cell is subjected to a second electrosensitising step, which sensitises the cell to ultrasound. As noted above, the pre-sensitising step is optional, and identical or similar results are obtained when this step is omitted in the following examples.

### Loading protocol

This section describes protocols for the loading and sensitisation of red blood cells by a combination of electrosensitisation (ES) followed by hypoosmotic dialysis loading (HD) overnight rest and further treatment of the cells by electrosensitisation. This combination is abbreviated as ES+HD+ES.

10 ml of peripheral venous blood is collected by venipuncture, into lithium heparin anticoagulant containing tubes, and mixed gently. The whole blood is then poured into a polypropylene tube and centrifuged at 300g for 15min at room temperature. The plasma and white blood cells (buffy coat) are removed.

1x phosphate buffered saline (PBS, made from Oxoid tablets code BR14a pH7.3) is added and the cells centrifuged at 700g for 5min. The supernatant is removed and the pellet of remaining cells resuspended in ice cold 1xPBS. The spin/wash procedure is then repeated once, and cells are suspended in ice-cold PBS at 6x10⁸ cells/ml.

Cells are then electrosensitised by dispensing 800µl of the RBC into sterile electroporation cuvettes, and placed on ice. To electrosensitise the cells, they are exposed to an electric field at 3.625kV/cm, 1 µF (2 pulses), in the absence of payload. The RBCs are then removed, and pooled in polypropylene tubes.

Cells are centrifuged once at 700g for 5min at room temperature (RT). The cells may be diluted in PBS/MgCl₂ (4mM). Cells are then resuspended in PBS/MgCl₂, and centrifuged at 700g for 5min, twice. Finally, cells are resuspended in PBS/MgCl₂, at approximately 7x10⁸c/ml, and rested for 30min at room temperature.

Cells are then loaded with payload by hypo-osmotic dialysis, according to a protocol adapted from Eichler *et al.,* (1986) Clin. Pharmacol. Ther. 40:300-303. Essentially the protocol is as follows:

### 1 REAGENTS AND BUFFERS:

Stock potassium phosphate buffer:
5mM K₂HPO₄ 3H₂O (FW 228.2g) ⇒ 1.141 g/L
5mM KH₂PO₄ (MW136.1g) ⇒0.68g/L
Stored at 4°C
Mix as follows:
For a pH7.4 K₂H/KH₂ phosphate buffer ⇒approx. 6.1:3.9 parts
Mix the 2 stock solutions as and when required

**Buffer #1 (isoosmotic PBS):**
pH7.4 K₂H/KH₂ phosphate buffer
150mM NaCl ⇒ 8.76g/L
Check and adjust pH (1M NaOH)

**Buffer #2 (dialysis buffer):**
pH7.4 K₂H/KH₂ phosphate buffer
Check and adjust pH (1M NaOH)

**Buffer #3 (resealing buffer)**
pH7.4 K₂H/KH₂ phosphate buffer
150mM NaCl ⇒ 8.76g/L
10mM glucose ⇒ 1.8g/L
Check and adjust pH (1 M NaOH)

### SpectraPor DIALYSIS TUBING PREPARATION:

1 The 12-14kDa MW cut off tubing, 0.32ml/cm, is used.
2 Preparation: heat at 80°C/30min in 1mM EDTA/2% sodium bicarbonate (Sigma). Rinse well, inside and outside, with ddH₂O.
3 Wash inside and outside with Buffer #1
4 Store submerged in a small amount of Buffer #1 if not used immediately.

### RBC PREPARATION:

1 Electrosensitised, rested RBC are washed in PBS twice at 700g for 5min.
2 For the final wash, cells are washed in buffer #1
3 The cells are manipulated as a suspension of packed cells following removal of final wash supernatants after centrifugation.

### CELL VOLUME IN TUBING:

1 Protocol recommends 60% haematocrit (HCT). The suspension of packed cells is approximately 75% HCT and is diluted accordingly.
2 Mix cells with the payload and buffer # 1, to give required final payload concentration and volume.

### 2 DIALYSIS:

1 The tubing is clipped to ensure that the surface area remains constant for the volume of cells.
2 Dialyse RBC (packed cell volume in buffer #1) against buffer #2 for 90min at 4°C.
3 Place membranes in 100-200ml buffer #2, (ensure that the membrane is immersed) in glass beaker with magnetic flea.
4 Place this beaker within another beaker, which contains ice, on the magnetic stirrer, cover with silver foil.
6 Warm up an aliquot of buffer #3 to 37°C.
7 Remove dialysis buffer, replace with the warm resealing buffer #3.
8 Place beaker with dialysis tubing and buffer #3 into a larger beaker anchored by water at 37°C, cover and leave for 15min.
9 Harvest cells into 12ml polypropylene tubes.
10 Wash x3 in ice cold resealing buffer #3 at 300g, 10 min 4°C.
11 Wash x1 in PBS/Mg/glucose and spin at 700g, 5min 4°C.
12 Count cells and resuspend at 7x10⁸c/ml, in PBS/Mg/glucose.
13 Store at 4°C overnight.

In the present example, dialysis is performed in the presence of 1.5mg of antibody per ml of cells. Cells are suspended at 7x10⁸ cells/ml.

When cells have been loaded they are then sensitised by exposure to electric pulses as follows:

### 3 ELECTROSENSITISATION

1 Following overnight storage, wash RBC once in PBS 700g, 5min 4°C.
2 Count cells and resuspend at 6x10⁸c/ml, in ice cold PBS.
3 Dispense 800µl of the RBC into sterile electroporation cuvettes (0.4cm gap).
4 Place on ice.
5 To electrosensitise: double pulse at 3.625kV/cm, 1 µF.
6 Harvest the RBC, pool in a polypropylene tube.
7 Centrifuge once at 700g for 5min room temperature (RT). The cells may be diluted in PBS/MgCl₂(4mM).
8 Resuspend in PBS/MgCl₂, centrifuge at 700g for 5min.
9 Repeat step 6
10 Resuspend in PBS/MgCl₂, at approximately 7x10⁸c/ml.
11 Rest the cells for 30min at RT.
12 Centrifuge once at 700g for 5min room temperature (RT). The cells may be diluted in PBS/MgCl₂/glucose.
13 Resuspend the cells in PBS/MgCl₂/glucose, centrifuge at 700g for 5min.
14 Repeat step 13.
15 Resuspend cells in PBS/MgCl₂/glucose at 7x10⁸c/ml.
16 Rest the cells in PBS/MgCl₂/glucose for 60min.

### I. Ultrasound-mediated release of antibody from vehicle

Antibody-loaded sensitised cells are then exposed to ultrasound at a distance of 1.3cm from the emitting surface of the ultrasound head. The intervening space is filled with the TMM as described above and 0.1 ml aliquots of 7x10⁸ cells/ml are exposed to ultrasound. In these studies a sheep anti-human von Willebrand factor antibody is employed as the payload in these studies. The amount of antibody in cells and released following treatment with ultrasound is quantified with an ELISA system, using standard protocols (as disclosed in for example, Harlow and Lane, Antibodies: a Laboratory Manual, (1988) Cold Spring Harbor, and Maniatis, T., Fritsch, E. F. and Sambrook, J. (1991), Molecular Cloning: A Laboratory Manual. Cold Spring Harbor, New York, Cold Spring Harbor Laboratory Press).

### I. Results

In the loading and sensitisation protocol, cells are loaded at a concentration of 1.1mg of antibody per ml of packed cell volume (PCV). 0.1ml aliquots of cells at 7x10⁸ cells/ml are exposed to ultrasound at intensities shown in Fig.9 and samples re analysed for cell lysis by direct counting. In addition, the amount of antibody released following treatment with ultrasound is determined by ELISA analysis of cell supernatants harvested following centrifugation. The results obtained are shown in Fig.9 and they demonstrate that cells were preferentially lysed at ultrasound power densities greater than 2W/cm². Control cells exhibit little or no effect when treated with ultrasound at these power densities. At and above 2W/cm² antibody payload is detected in supernatants harvested following ultrasound treatment. In addition, when the total amount of antibody released from the cells using ultrasound is compared with that released following hypotonic lysis in 0.01% (v/v) Triton X100 it is found that 77% of the total antibody is released in the former. The remainder could be found in debris that is recovered by centrifugation following ultrasound treatment.

The results demonstrate that ultrasound-mediated release of payload can be achieved using relatively low intensity ultrasound and using conditions which have little or no effect on normal erythrocytes. The results also demonstrate ultrasound-mediated release of payload at a depth of 1.3cm and thereby demonstrating one of the major advantages associated with the use of ultrasound as the releasing stimulus of penetration to depth in tissues. Since all of the antibody incorporated into the ultrasound treatment experiments can be recovered as shown using an ELISA based on payload functionality, this suggests that the ultrasound has no detrimental effect on that functionality

### II. Ultrasound-mediated release of enzyme (β-galactosidase) from vehicle

Cells are harvested, primed by exposure to electric pulses and loaded with β-galactosidase (from *Escherichia coli*, Sigma) as described above for antibody loading. Cells are subsequently exposed to sensitising electric pulsing and exposed to ultrasound at a concentration of 7×10⁸ cells/ml in the TMM system as described above for the antibody-loaded vehicle. Lysates obtained following exposure of the loaded and sensitised vehicle to ultrasound are assayed for β-galactosidase activity at 37°C using the colorimetric substrate p-nitrophenyl-β-D-galactoside (5mM in 50mM phosphate buffer, pH 7.0). The concentration of p-nitrophenol is determined spectrophotometrically at 450nm and activity is expressed as µmoles of p-nitrophenol produced per minute per ml of sample. Release of enzyme in samples harvested following treatment with ultrasound is expressed as a percentage relative to the amount of enzyme contained in the cells prior to treatment. The latter is determined by measuring the amount of enzyme released from the cells following lysis by freeze-thaw in 5mM phosphate buffer, pH 7.2.

### II. Results

In these experiments loaded cells contain approximately Img of enzyme per ml of packed cell volume. The results obtained following treatment of these preparations with ultrasound are shown in Fig. 10. Samples are treated at the indicated power densities as shown and samples are analysed for cell lysis by cell counting. Lysis increases with increasing power density up to a maximum at about 3W/cm². Exposure of control normal cells to similar ultrasound conditions has little or no effect on cell lysis and this is confirmed by the absence of hemoglobin in supernatents following removal of cells by centrifugation. When supernatants are harvested by centrifugation, following exposure of the sensitised and loaded cells to ultrasound and analysed for enzyme content, it is found that increasing amounts of enzyme are released with increasing power density up to a maximum at 3W/cm². The results demonstrate that enzyme is released from the vehicle following exposure to low intensity ultrasound. Ultrasound-mediated release of the payload is achieved at 1.3cm from the emitting ultrasound head, indicating that the use of ultrasound for this purpose offers the advantage of penetration to depth in tissues. In addition, since 100% recovery of the enzyme released is achieved (between 2.5-3W/cm²), the ultrasound stimulus resulting in release of enzyme has no detrimental effect on the functionality of the released payload.

### III. Ultrasound-mediated release of oligonucleotide from vehicle

Cells are harvested and primed by exposure to electric pulses as described above. Cells are then loaded using the hypoosmotic dialysis procedure described above for antibody loading and a 300µg quantity of oligonucleotide (tamara labelled random 30-mer supplied by Oswel, UK) is mixed with 250µl of packed cells. Samples are then subjected to electrosensitisation electric pulses and subsequently suspended in PBS/MgCl₂/glucose at a concentration of 7×10⁸ cells/ml. Samples are exposed to ultrasound using the TMM system described above for antibody and enzyme release and the amount of oligonucleotide released is determined using a spectrofluorimeter (Shimadzu) with excitation set at 540nm and emission set at 590nm. A standard curve is constructed for quantitative determinations and extraction efficiencies are taken into account.

### III. Results

In these experiments the maximum amount of oligonucleotide loaded is approximately 300µg of oligonucleotide per ml of packed cell volume. The results obtained following treatment of these loaded preparations with ultrasound are shown in Fig.11. As with the above two examples, cell lysis of the sensitised and loaded preparation occurs between 2 and 3 W/cm². Under these ultrasound conditions there is little or no effect on control erythroyctes. In addition, oligonucleotide begins to appear in harvested supernatants between 2 and 3 W/cm² demonstrating ultrasound-mediated release of oligonucleotide payload from the vehicle. These results also demonstrate release of the payload from the vehicle when the target cells are at a distance of 1.3cm from the emitting surface of the ultrasound head and this again indicates the advantage of penetration to depth in tissues associated with the use of ultrasound as a release stimulus.

### EXAMPLE 11

### Ultrasound-mediated release of antibody payload in a perfused rat kidney system

Although it is demonstrated in the above examples that a variety of payloads can be released in a TMM system it is also of interest to demonstrate release of a functional payload in a tissue. To this end human erythrocytes are loaded with FITC-labelled anti-von Willebrand factor antibody and sensitised as described in Example 10 above. These cells are then administered to PBS-perfused rat kidneys and treatment with ultrasound is carried out according to the following protocol:
Perfuse the rat through the heart with PBS/EDTA until the kidney is clear of blood
Remove the dorsal aorta from the heart and insert a gavage needle into the vessel.
Tie the needle to the dorsal aorta using suture.
Close the dorsal aorta and posterior vena cava just after the junction leading to the kidney.
Close the left adrenal artery and vein and both anterior mesenteric and coeliac arteries
Close the ureter and the left iliolumbar artery and vein.
Create an exit point by inserting a gavage needle into the vena cava just before the liver. Tie the needle using suture.
Flush with 10ml PBS/4mM Mg/10mM glucose and check for any leakage.
Block the exit point by inserting 2ml syringe into the gavage needle to establish positive pressure.
Load 1ml of 7x10⁸ cells /ml through the dorsal aorta into the kidney.
Treat with U/S using 1MHz probe.
Incubate the treated kidney for one hour
Remove the 2ml syringe and flush through with 2ml PBS/Mg/glucose
Collect the flush through for cell counting and ELISA
Flush with 50ml of PBS/EDTA
Flush with 20ml of 4% neutral buffered formalin (NBF)
Remove the U/S-treated kidney and cut it into two halfs and fix in NBF
Prepare tissue sections (12µm) and stain using Vectastain ABC kit (Vecta Labs) as outlined in the manufacturer's instructions.

### RESULTS 11

As shown in Figure 12, kidney endothelial cells in glomeruli are labeled with the FITC conjugated anti-vWF antibody after ultrasound treatment to release the antibody (+U/S). In the absence of ultrasound treatment, no staining is observed ([-U/S]). The results demonstrate that low intensity ultrasound may be used to effect release of functional antibody from the loaded and sensitised vehicle in a perfused tissue system.

### EXAMPLE 12

### Circulation of normal, electrosensitised, glutaraldehyde-treated and PEGylated normal rabbit erythrocytes in vivo.

Since it is widely known that damaged erythrocytes are rapidly removed from circulation by macrophages of the reticulo-endothelial system (DeLoach and Barton, 1981, *Am*. *J. Vet*. *Res*. 42, 1971-1974), it is of interest to determine whether or not electrosensitised erythrocytes remain in circulation. As mentioned above it has also been demonstrated that PEGylation has the ability to protect foreign entities from recognition by reticulo-endothelial system and to this end it was decided to PEGylate normal erythrocytes and demonstrate that the modification prevented recognition by that system. In order to examine the above, it was decided to examine circulation of normal, electrosensitised, PEGylated and glutaraldehyde-treated rabbit erythrocytes *in vivo.* The latter treatment is chosen as a positive control for sequestration by the reticulo-endothelial system since it has been shown to promote targeting by the RES and consequential rapid uptake by the liver/spleen. In these studies circulation of the labelled vehicle is monitored by firstly labelling the cells with ⁹⁹Tc and subsequently monitoring the fate of that label using a gamma camera and acquiring full body scans of the recipient animal.

To the above ends peripheral rabbit venous blood is obtained by venipuncture, and added to tubes containing lithium heparin anti-coagulant. The donor rabbits are male and female New Zealand white rabbits which weighed between 3kg and 4kg. Whole blood is centrifuged at 300g for 15 minutes at room temperature. Plasma and white cells are removed and the cell pellet resuspended to 10ml in cold PBS. Cells are washed twice at 800xg for 3 minutes and the pellets resuspended with cold PBS. Erythrocytes are diluted in PBS and counted as described above. Peripheral venous blood collected as described previously is aliquoted into Eppendorf tubes and centrifuged at 800g for 3 minutes at room temperature. Plasma is harvested and stored in Eppendorf tubes at room temperature until required.

In cases where cells are electrosensitised, RBC are resuspended at between 7-8x10⁸ cells/ml in cold PBS. Seven hundred µl of the cell suspension and 100µl of cold PBS are added to sterile electroporation cuvettes. Cuvettes are mixed and pulsed twice at 1.45kV and 1µF with a BioRad electroporator. Cells are harvested into Eppendorf tubes, centrifuged once then washed twice with 1ml of cold PBS at 800xg for 3 minutes. The final pellet is resuspended with 760 µl of cold PBS/ 4mM MgCl₂. Cells are left on ice for 30 minutes. Next the cells are washed in a buffer (145mM NaCl, 2.4mM KCI, 7.6mM Na₂HPO₄2H₂O, 2.4mM NaH₂PO₄2H₂O, 4mM MgCl₂, 10mM glucose). Ultrasound sensitivity following electrosensitisation is verified by exposing the cells to 3 W/cm² for 35sec. on the TMM system and using the 1MHz ultrasound head.

In cases where cells are PEGylated the procedure is that described in Scott *et al* PNAS 94:7566 1997. Cells are washed at 800xg for 3 minutes, resuspended in cold PBS, pooled and counted. Counts are typically between 7-8x10⁸ cells/ml. In order to determine whether PEGylation is successful 1µl of the appropriate agglutinating IgM antibody (Lorne Laboratories Ltd) is added to 10µl of a 1:2 dilution of PEGylated RBC and to 10µl of a 1:2 dilution of non-PEGylated RBC. The glass slides are rocked gently, agglutination is observed in non-PEGylated samples but not in the PEGylated samples.

Cells are glutaraldehyde treated as follows: 1 ml of packed cells are technetium labelled as described above. 0.5 ml of glutaraldehyde at 2.5% v/v was then added to the labelled cells, and the preparation allowed to stand for 5 minutes at room temperature. Cells are then washed twice with PBS.

In all cases, preparations of cells are ⁹⁹Tc labelled for monitoring purposes *in vivo.* Essentially one ml of the prepared RBC (between 7-8x10⁸c/ml) and 0.5ml of autologous plasma are mixed and then injected into the technetium kit as per the kit protocol (Mallinckrodt UK Ltd, Bichester, UK). Following labelling, the contents of the kit are harvested and the cells washed 3 times in PBS at 800xg for 3 minutes. Supernatants are retained to check the levels of radioactivity. The final cell pellets are resuspended in 1ml of PBS and the cells counted. The cells are then injected into the right ear of the rabbits (male or female New Zealand white rabbits, which weighed between 3 and 4kg). Following injection, the fate of circulating RBC is monitored with a gamma camera and whole body images are acquired at times ranging from time zero to 20 minutes.

### RESULTS 12

Results are presented as four panels in Fig.13 A,B,C and D representing whole body scans of rabbits injected with electrosensitised, normal, gluraraldehyde treated normal and PEGylated normal rabbit erythrocytes, respectively. In panels A to C, whole body scans are captured at the times indicated in Fig. 13 over a period of time zero to 20 minutes. In Fig.13, panel B the distribution of labelled normal erythrocytes is seen over this time period and represents a normal distribution. Liver, kidneys and ventral line vasculature to fermorals are clearly imaged and no preferential accumulation in the liver-spleen is seen in the images. The distribution of electrosensitised rabbit cells (Fig. 13, Panel A) over the scanning time period is similar to that exhibited by normal cells and from this one can conclude that the half life of the sensitised cells mimics that of normal cells. No preferential accumulation in the liver or spleen over that exhibited by the normal cells is apparent. As mentioned above, glutaraldehyde treatment of erythroyctes promotes recognition by the reticulo-endothelial system with the consequence of rapid sequestration by the liver and spleen. Fig. 13, panel C clearly shows dramatic premature accumulation of the radioactively labelled glutaraldehyde-treated cells in the liver and spleen of the recipient rabbit. On the basis of comparison between the distribution of normal, electrosensitised and glutaraldehyde-treated normal cells over the scanning time period it is clear that the circulation of electrosensitised cells is not compromised by any modification resulting from the electrosensitisation procedure. The results suggest that the use of electrosensitised erythrocytes as a vehicle for payloads offers the advantage over competing technologies (e.g. liposome technology) in that they are not recognised by the reticulo-endothelial system, thereby offering prolonged circulation times *in vivo*.

In addition, when PEGylated normal erythrocytes are introduced into a recipient animal, it is found that their distribution during circulation over the scanning time examined is normal (Fig. 13, Panel D). In this case scan capture is more frequent as indicated, but the overall scan time again ranged from time zero to 20 minutes. This latter result suggests that PEGylation of erythrocytes does not cause damage which results in recognition of the modified cells by the reticulo-endothelial system and further suggests that if our carrier erythrocyte technology requires PEGylation for what ever reason, then it may be applied without negative consequence in terms of premature removal from circulation.

### EXAMPLE 13

### The effect of PEGylation on circulation of human erythroyctes in the rabbit

As mentioned above PEGylation of foreign entities aids in preventing recognition by macrophages of the reticulo-endothelial system. In order to determine whether or not PEGylation facilitates prolonged circulation of species-heterologous erythroyctes it was decided to examine circulation of normal, PEGylated normal and PEGylated electrosensitised human erythroyctes in rabbits using the ⁹⁹Tc-based monitoring method described above.

To this end, peripheral venous blood is obtained by venipuncture, and added to 10ml blood collection tubes containing lithium heparin anti-coagulant. Donor phenotype is determined by agglutination with antisera from Lorne Laboratories Ltd, (Twyford, UK). Aliquots (1ml) of whole blood was added to Eppendorf tubes and centrifuged at 300g in a centrifuge (Heraeus Biofuge *pico*), for 15 minutes at room temperature. Plasma and white cells are removed and the cell pellet is resuspended to 1 ml in cold (4°C) phosphate buffered saline (PBS, Oxoid, Basingstoke, UK). Cells are washed at twice at 800g for 3 minutes, in cold PBS. Pellets are resuspended to a final volume of 12ml with cold PBS. Erythrocytes/red blood cells (RBC) are diluted 1 in 200 and counted with a haemocytometer. Peripheral venous blood collected as described previously is aliquoted in Eppendorf tubes and centrifuged at 800g for 3 minutes at room temperature. Plasma is harvested and stored in Eppendorf tubes at room temperature until required. In cases where electrosensitisation and PEGylation are required, these were performed as described above for Example 12.

One ml of the prepared RBC (between 7-8x10⁸c/ml) and 0.5ml of autologous plasma are mixed and then injected into the technetium kit as per the kit protocol (Mallinckrodt UK Ltd, Bichester, UK). Following labelling, the contents of the kit are harvested and the cells washed 3 times in PBS at 800xg for 3 minutes. Supernatants are retained to check the levels of radioactivity. The final cell pellets re resuspended in 1ml of PBS (with the exception of (+) EP cells which are resuspended in 0.5 ml of PBS) and the cells counted. The cells are then injected into the right ear of the rabbits (male or female New Zealand white rabbits, which weighed between 3 and 4kg). Following injection, the fate of circulating RBC is monitored with a gamma camera. The accumulated images are analysed by specialised computer packages. The circulation time of RBC is analysed by monitoring circulating labelled erythroyctes in the heart.

### RESULTS 13

The results obtained from these experiments are shown in Fig.14. When labelled normal, PEGylated normal and PEGylated electrosensitised cells are introduced into recipient rabbits, the proportion of labelled cells remaining in circulation drops to approximately 35% within 1 minute. However, at time intervals following that stage, the advantage associated with PEGylation becomes apparent particularly at 10minutes. At 10 minutes the unmodified normal human cells continue to decrease in circulation whereas survival of the PEGylated normal and PEGylated human cells in circulation is enhanced.

The results suggest that if species-heterologous or modified autologous/homologous sensitised erythroyctes are to be employed as a delivery vehicle, recognition by the macrophages of the reticulo-endothelial system and consequential removal from circulation can, at least in part, be prevented by PEGylation.

### EXAMPLE 14

Since the above studies demonstrate that sensitised rabbit erythroyctes are stable during circulation *in vivo* it was decided to confirm these results using an alternative labelling method and monitoring system. It has been shown that erythrocytes, may be conveniently labelled using the fluorogenic label PKH-26 which incorporates into the membrane of the cell (Selzak & Horan, 1989, Blood, 74, 2172-2172). Cells may therefore be introduced into a recipient animal and monitored using flow cytometry. If this system can be employed to monitor normal (autologous, heterologous rabbit) and electrosensitised rabbit cells during circulation *in vivo* it will circumvent problems associated with the relatively short half life of ⁹⁹TC.

Cells are harvested and where required, electrosensitised as described above in Example 12. Cells are then PKH-26 labelled as follows:
1 - Remove 5ml of blood from rabbit ear vein
2 - Wash twice to remove buffy coat in saline and process cells as required for treatment
3 - Re-suspend cells in saline to give a packed cell volume of ~0.25ml
4 - Centrifuge & remove supernatant
5 - Add 1ml of PKH-26 labelling kit buffer C (Sigma #MINI-26) & resuspend cells
6 - To 1ml of buffer C add 4µl of 1mM PKH-26 solution, mix well & add to cell suspension
7 - Mix tube by gentle inversion for 5 minutes
8 - Spin for 10'
9 - Remove supernatant & wash 3 times in saline
10 - Count cells & resuspend in 1ml of saline
11 - Verify cell labelling using flow cytometry

Following labelling cell preparations (0.5ml packed cells) are injected into the rabbit ear vein (recipient animal weighs between 3 and 4kg). At the indicated times 50µl samples are collected into 1ml of heparin-containing saline. Samples are subsequently analysed using flow cytometry and the percentage of labelled cells in circulation is determined.

### RESULTS 14

Rabbits are injected with PKH-26 labelled rabbit normal autologous, normal heterologous and electrosensitised cells. PKH-26 labelled human erythrocytes are used as a control for reticulo-endothelial scavanging. Samples are harvested at the indicated times and the labelled cells in circulation expressed as a percentage of the amount detected at time zero are detected by flow cytometry. The results obtained are shown in Fig.15 and they demonstrate that PKH-26 labelled normal autologous and heterologous rabbit cells circulate normally (see Fig.15 for predicted status of normal cells based on published T_{1/2} for normal rabbit erythrocytes [from Vomel & Platt, 1981, Mech. Ageing Dev. 17, 261-266]). In addition, PKH-26 labelled electrosensitised cells are also shown to circulate with pharmacokinetics similar to those exhibited by normal cells (Fig.15). On the other hand human cells are rapidly cleared from circulation. The results confirm our earlier findings that electiosensitised cells circulate with pharmacokinetics similar to those exhibited by normal circulating rabbit cells.

### EXAMPLE 15

### Survival of loaded and electrosensitised erythroyctes in vivo

As shown above, electrosensitised cells are stable during circulation *in vivo.* It is also of interest to determine whether or not cells which are electrosensitive and also processed through a loading protocol such as hypotonic dialysis loading, remain intact during circulation. It was therefore decided to produce ultrasound sensitive erythroyctes loaded with antibody (rabbit anti-human IgG) and introduce them into an animal. The cells are PKH labelled and their circulation *in vivo* is monitored using flow cytometry as described above.

Rabbit erythrocytes are harvested and washed by centrifugation in PBS. Erythroyctes are loaded and sensitised by a modification of the procedure devised for human cells and described in detail as follows:

### Buffers and Solutions:

PBS: Phosphate buffered saline tablets (Oxoid: Code BR14a) made up as per instructions

PBS-Glutathione: Above supplemented with 0.5mM reduced glutathione

PBS-MgCl₂-Glutathione: PBS (above) supplemented with 4mM MgCl₂ and 0.5mm reduced glutathione.

Dialysis buffer (hyptonic): 12.5mM K₂HPO₄/KH₂PO₄ containing 0.5 mM reduced glutathione, pH 7.4

PIGPA: 5mM adenine, 100mM inosine, 2mM ATP, 100mM sodium pyruvate, 100tnM glucose, 4mM Mg Cl₂, 194mM NaCl, 1.6mM KCl, 35mM NaH₂PO₄, pH 7.4

BAX/0.5mM Glutathione: PBS (as above) supplemented with 5mM adenosine, 5mM glucose, 5mM MgCl₂ and 0.5mM reduced glutathione, pH 7.4.

### Dialysis Tubing Preparation:

Dialysis Tubing: Spectra/Por® Membrane MWCO: 12-14,000 No.2
Flat Width: 10+/- 1mm
Diameter: 6.4 mm

### Method:

### Day one

1 - Collect 10ml of blood from rabbit ear vein into 10ml heparinised tube
2 - Transfer blood to 15ml tube & spin at 3,000rpm for 3' at RT°
3 - Remove plasma & buffy coat
4 - Add equal volume of PBS, re-suspend & spin at 2,000rpm for 15' RT°
5 - Repeat step 4
6 - Aliquot 0.5ml of packed cells plus 1mg of Rb α-Hu IgG into the dialysis tubing.
7 - Place tubing into a 3ml electroporation cuvette & fill the cuvette with PBS-GSH.
8 - Electroporate at RT°, 5kV/cm, 3µF, double pulse.
9 - Remove dialysis tubing immediately & place into 100ml of dialysis buffer on ice.
   Dialyse with stirring for 30'.
10- Transfer tubing to tubes containing 30ml of PBS-MgCl₂ & incubate for 11' at 37°C with intermittent agitation of tubes.
11- Transfer the contents to 1.5ml eppendorf tube & measure the volume. Add PIGPA to the tube at 1/10^{th} the volume & incubate at 37°C for 30'.
12- Transfer to a 15ml tube & bring the volume to 2ml with Bax buffer at RT.
13- Centrifuge cells at 900rpm for 5'.
14- Resuspend cells in 2ml Bax buffer & store O/N at 4°C

### Day two

1- Following O/N storage centrifuge samples at 900rpm for 5' at 4°C
2- Remove supernatant and resuspend to 4ml with Bax buffer & wash twice at 900rpm for 5', followed by 1 x 1400rpm centrifugation for 5'
3- Resuspend samples initially in 2ml of Bax buffer & pool samples into a 15ml tube on ice & remove a small sample. Adjust volume of the sample to give a cell concentration of 7 x 10⁸cells/ml)
4- Verify ultrasound sensitivity (1MHz, 1.25W/cm²,15 seconds)

The loaded and sensitised cells are then labelled with PKH-26 as described above and again ultrasound sensitivity is verified. In these experiments 1x10⁹ cells are injected into a rabbit (3kg) which is anaesthetised by injection with a 0.3ml Vetalar/0.2ml Rompun mixture into the left ear vein. A 50µl sample is taken prior to injection and placed in 450µl of PBS containing heparin. Subsequent samples are taken at the times indicated in a similar manner post injection of labelled cells and samples are analysed by flow cytometry.

### RESULTS 15

The results obtained are shown in Fig. 16 and they demonstrate that counts of labelled cells remain above background throughout the period examined. The line indicated by upright triangles is simply for reference to the counts detected in the blood sample taken prior to injection of the labelled cells. The results demonstrate that approximately 70% of the introduced cells remain at two hours. It should be noted that if these cells are being recognised by the reticulo-endothelial system, clearance should occur within 5 minutes. Since this does not occur the results clearly demonstrate that the loaded and sensitised vehicle is relatively stable during circulation *in vivo.*

### EXAMPLE 16

### Ultrasound-mediated release of payload from the loaded, sensitised vehicle in a circulating system at 37°C and at high hematocrit (Hct.)

In the above studies it is shown that human erythroyctes can be sensitised to low intensity ultrasound and ultrasound-mediated disruption and/or payload release can be achieved in *vitro* and in an *ex vivo* perfused rat kidney system. In all of those systems disruption and/or payload release is demonstrated at 7x10⁸ cells/ml which is approximately equivalent to a 5% Hct. In addition, those studies are performed at room temperature. Since Example 12 demonstrates that human cells are rapidly cleared from circulation in an animal model system it is of interest to demonstrate that sensitivity in terms of payload release can be retained at 37°C and at higher Hct. It is also of interest to determine whether or not this occurs while the target cells are moving through a circulation system in much the same as those circulating *in vivo.*

To these ends human erythrocytes are harvested and loaded with anti-von Willebrand factor antibody as described for Example 10. Following sensitisation cells re mixed together with normal washed human cells in the proportions of one part 7x10⁸ cells/ml and four parts 4x10⁹ cells/ml. The mixture is introduced into a circulating system consisting of a cylindrical reservoir filled with PBS and maintained at 37°C by circulation. The bottom of the cylinder consists of a light polyethylene sheet through which ultrasound is delivered. The blood is circulated through C-flex tubing (internal diameter 4mm) which passes through the thermostated buffer and the target area of the C-flex tubing is positioned at a distance of 1.3cm from the ultrasound-emitting head. Blood is circulated through the system at a rate of 14.5ml/min. During exposure to ultrasound (5W/cm² at 1MHz for indicated times), samples are harvested from the system and supernatants are harvested by centrifugation. These are then assayed for antibody using an ELISA assay as described above. The control in these experiments consists of loaded and sensitised cells circulated through the system in the absence of ultrasound. It is also important to note that circulation of normal cells through the system while ultrasound is being delivered results in no apparent damage as determined by the lack of hemoglobin in supernatants following treatment.

### RESULTS 16

The results are shown in Fig.17 and they demonstrate that detectable quantities of antibody are released from the vehicle between 2 and 5 minutes treatment with ultrasound. Little or no antibody can be detected in control samples which consist of the loaded and sensitised cells circulated through the system in the absence of ultrasound. The results demonstrate that the ultrasound-sensitisation phenomenon is intact at 37°C and ultrasound-mediated release of payload is achieved at high hematocrit (Hct.) and in a mobile target system.

### EXAMPLE 17

### Circulation of Payload and Detection Limits

Since it has been demonstrated in the above examples that loaded and sensitised rabbit erythrocytes are relatively stable *in vitro* it was decided to examine ultrasound-mediated release of payload in the rabbit. In these studies rabbit anti human IgG is chosen as the payload and quantified using an ELISA system based on recognition of human IgG. Prior to embarking on *in vivo* studies involving release of antibody from the vehicle, it was decided to examine circulation of the payload alone in order to demonstrate that it is not prematurely removed from circulation. This was also carried out in order to determine the detection limits of the antibody in plasma during circulation.

To this end rabbits (approx. 3kg) are anaesthetised by injection with 0.3ml Vetlar and 0.2ml Rompun into the ear vein. Antibody is injected into the recipient rabbits at a concentration of 0.5mg/kg. A 50µl sample of blood is harvested prior to injection of antibody and mixed together with 450µl of PBC containing heparin. Following administration of antibody 50µl samples are harvested at the indicated times and mixed together with 450µl of PBS containing heparin. Samples are then centrifuged at 2000rpm using a microfuge and the supernatants are aliquoted into 150µl quantities for storage at - 80°C. Samples are analysed for antibody content using an ELISA method based on recognition of human IgG.

### RESULTS 17

When harvested samples are analysed for rabbit anti-human IgG activity by ELISA the profile shown in Fig.18 is obtained. The results demonstrate that the introduced antibody remains at detectable levels in circulation for up to 21 days and this would be well within the required time frame for ultrasound-mediated release studies. In addition the lowest detectable concentration of 0.6µg/ml at day 21 suggests that loaded cells containing 10µg of antibody are sufficient for the ultrasound-mediated release studies.

### EXAMPLE 18

### Ultrasound-mediated release of payload from the erythrocyte delivery vehicle in vivo.

In order to demonstrate that ultrasound-mediated release of payload from the sensitised and loaded vehicle can be effected *in vivo* it was decided to sensitise and load rabbit erythrocytes with rabbit anti-human IgG. This is performed as described above for Example 15. Ultrasound sensitivity is confirmed prior to use and the cell population is shown to contain approximately 200µg of antibody/ml of packed cells.

The recipient rabbit (3kg) is anaesthetised by injecting 0.45ml of Vetalar and 0.3ml of Rompun sub-cutaneously into the back of the neck. Hair is removed from the abdominal area over the liver. Anaesthesia is maintained by placing the animal on 2% isofluorane delivered via a face mask. A pre-injection sample of blood (50µl) is taken and placed in 450µl of PBS containing heparin. 0.5ml of packed cells are then injected into the animal through the ear vein. The animal is rested for 10 minutes and another sample of blood is harvested. Ultrasound contact gel is liberally applied to the shaven area to mediate contact with a 1MHz ultrasound head. Six 4-minute ultrasound treatments are applied on continuous wave delivery at 4W/cm² with each treatment interrupted by 30 seconds within which a sample of blood is harvested. Following the six treatments the animal is rested for 20 minutes and two blood samples are taken at 10 minute intervals. Treatment is reinitiated at 60 minutes again at 4-minute intervals with 30-second rest intervals within which blood samples are taken. The animal is again rested after six treatments for a period of 30 minutes during which samples of blood are harvested at 10-minute intervals. The control animal consists of an animal into which a similar quantity of cells are injected and ultrasound treatment is withheld. Both animals are euthenised using pentobarbitone following treatment. Supernatants are harvested by centrifugation from all samples and subsequently assayed by ELISA to detect circulating rabbit anti-human IgG.

### RESULTS 18

The results obtained during these experiments are shown in Fig.19. Ultrasound treatments are indicated by the solid arrows and the dotted line traversing the lower portion of the curve represents the pre-injection background signal received from the ELISA. The results obtained from the ultrasound treated animal demonstrate the presence of antibody in circulation following 16 minutes treatment with ultrasound. After this period and during the rest period the amount of antibody in circulation decreases. However following the second phase of treatments initiated at 60 minutes the antibody concentration again begns to rise. In the control animal the level of antibody in circulation does not exhibit this pattern. It is interesting to note that the level of antibody in the first peak appearing in the ultrasound-treated animal accounts for approximately 16-20% of the total antibody contained in vehicle injected into the animal. This suggests that the remainder of antibody-containing vehicle supplies a sufficient reservoir for release as indicated by the second peak at 120 minutes. These results demonstrate ultrasound-mediated release of a payload from the sensitised and loaded vehicle *in vivo.*

## Claims

1. Use of an electric field under *in vitro* or *ex vivo* conditions for sensitising a red blood cell to ultrasound.

2. Use according to Claim 1, in which the electric field has sufficient energy to electrosensitise the cell.

3. Use according to Claim 1 or 2, in which the electrosensitised red blood cell may be selectively disrupted using ultrasound.

4. Use according to any preceding claim, in which the electric field has a strength from about 0.1kV/cm to about 10 kV/cm under *in vitro* conditions.

5. Use according to any preceding claim, in which the electrosensitisation comprises the step of applying an electric pulse to a red blood cell.

6. Use according to Claim 5, in which the electric pulse is applied for between 1µs and 100 milliseconds.

7. Use according to any preceding claim, in which the ultrasound is selected from the group consisting of: diagnostic ultrasound, therapeutic ultrasound and a combination of diagnostic and therapeutic ultrasound.

8. Use according to any preceding claim, in which the ultrasound is applied under in *vitro* or *ex vivo* conditions at a power level of from about 0.05W/cm² to about 100W/cm².

9. Use according to any preceding claim, further comprising the step of loading the red blood cell with an agent under *in vitro* or *ex vivo* conditions.

10. Use according to Claim 9, in which the loading is performed by a procedure selected from a group consisting of electroporation, sonoporation, microinjection, membrane intercalation, microparticle bombardment, lipid-mediated transfection, viral infection, osmosis, osmotic pulsing, diffusion, endocytosis, and crosslinking to a red blood cell surface component.

11. Use according to Claim 9 or 10, in which the loading of the agent is substantially simultaneous with the sensitisation of the red blood cell.

12. Use according to Claim 9 or 10, in which the sensitisation of the red blood cell precedes the loading of the agent.

13. Use according to Claim 9 or 10, in which the loading of the agent precedes the sensitisation of the red blood cell.

14. Use according to any of Claims 9 to 13, in which the agent is selected from a group consisting of a biologically active molecule, a protein, a polypeptide, a peptide, a nucleic acid, a virus, a virus-like particle, a nucleotide, a ribonucleotide, a deoxyribonucleotide, a modified deoxyribonucleotide, a heteroduplex, a nanoparticle, a synthetic analogue of a nucleotide, a synthetic analogue of a ribonucleotide, a modified nucleotide, a modified ribonucleotide, an amino acid, an amino acid analogue, a modified amino acid, a modified amino acid analogue, a steroid, a proteoglycan, a lipid, a carbohydrate, and mixtures, fusions, combinations or conjugates of the above.

15. Use according to any of Claims 9 to 14, in which the agent is conjugated to, fused to, mixed with or combined with an imaging agent.

16. Use according to any preceding claim, in which the red blood cell is PEGylated.

17. Use of ultrasound to disrupt an electrosensitised red blood cell wherein both the electrosensitisation and ultrasound disruption are carried out under *in vitro* or *ex vivo* conditions.

18. Use according to Claim 17, in which the red blood cell has been electrosensitised by being exposed to an electric field, including an electric pulse.

19. Use according to Claim 17 or 18, in which the ultrasound is selected from the group consisting of: diagnostic ultrasound, therapeutic ultrasound and a combination of diagnostic and therapeutic ultrasound.

20. Use according to Claim 17, 18 or 19, in which the ultrasound is applied at a power level of from about 0.05W/cm² to about 100W/cm².

21. Use according to any of Claims 17 to 20, in which the red blood cell is loaded with an agent under *in vitro* or *ex vivo* conditions.

22. Use according to Claim 21, in which the ultrasound disruption of the red blood cell releases the agent.

23. An *in vitro* or *ex vivo* method of selectively disrupting a red blood cell, the method comprising the steps of:
(a) electrosensitising the red blood cell by exposing the red blood cell to an electric field; and
(b) disrupting the red blood cell by subjecting the red blood cell to ultrasound.

24. A method according to Claim 23, further comprising loading the red blood cell with an agent under *in vitro* or *ex vivo* conditions.

25. A *in vitro* or *ex-vivo* method for selectively releasing an agent from a red blood cell comprising the steps of:
(a) loading a red blood cell with an agent;
(b) electrosensitising the red blood cell; and
(c) causing the agent to be released from the sensitised red blood cell by applying ultrasound at a frequency and energy sufficient to cause disruption of the red blood cell but insufficient to cause disruption of unsensitised red blood cells.

26. An *in vitro* or *ex vivo* method for providing a delivery vehicle suitable for delivery of an agent to a vertebrate, comprising the steps of:
(a) loading a red blood cell with an agent; and, in a separate step,
(b) exposing the red blood cell to an electric field to render it susceptible to disruption by ultrasound.

27. A method according to Claim 25, comprising subjecting the red blood cell to an electric field.

28. A method according to Claim 27, in which the electric field has sufficient energy to electrosensitise the cell.

29. A method according to Claim 23 to 28, in which the electric field has a strength from about 0.1 kV/cm to about 10 kV/cm under *in vitro* conditions.

30. A method according to any of Claims 23 to 29, in which the electrosensitised red blood cell may be selectively disrupted using ultrasound.

31. A method according to any of Claims 23 to 30, in which the electrosensitisation comprises the step of applying an electric pulse to a red blood cell.

32. A method according to Claim 31, in which the electric pulse is applied for between 1µs and 100 milliseconds.

33. A method according to any of Claims 23 to 32, in which the ultrasound is selected from the group consisting of: diagnostic ultrasound, therapeutic ultrasound and a combination of diagnostic and therapeutic ultrasound.

34. A method according to any of Claims 23 to 33, in which the ultrasound is applied at a power level of from about 0.05W/cm² to about 100W/cm².

35. A method according to any of Claims 24 to 34, in which the loading is performed by a procedure selected from a group consisting of electroporation, sonoporation, microinjection, membrane intercalation, microparticle bombardment, lipid-mediated transfection, viral infection, osmosis, osmotic pulsing, diffusion, endocytosis, and crosslinking to a red blood cell surface component.

36. A method according to any of Claims 24 to 35, in which the loading of the agent is substantially simultaneous with the sensitisation of the red blood cell.

37. A method according to any of Claims 24 to 35, in which the sensitisation of the red blood cell precedes the loading of the agent.

38. A method according to any of Claims 24 to 35, in which the loading of the agent precedes the sensitisation of the red blood cell.

39. A method according to any of Claims 24 to 38, in which the agent is selected from a group consisting of a biologically active molecule, a protein, a polypeptide, a peptide, a nucleic acid, a virus, a virus-like particle, a nucleotide, a ribonucleotide, a deoxyribonucleotide, a modified deoxyribonucleotide, a heteroduplex, a nanoparticle, a synthetic analogue of a nucleotide, a synthetic analogue of a ribonucleotide, a modified nucleotide, a modified ribonucleotide, an amino acid, an amino acid analogue, a modified amino acid, a modified amino acid analogue, a steroid, a proteoglycan, a lipid, a carbohydrate, and mixtures, fusions, combinations or conjugates of the above.

40. A method according to any of Claims 24 to 39, in which the agent is conjugated to, fused to, mixed with or combined with an imaging agent.

41. A method according to any of Claims 23 to 40, in which the red blood cell is PEGylated.

42. An *in vitro* or *ex vivo* method of electrosensitising a red blood cell to disruption by ultrasound, the method comprising the step of:
subjecting the red blood cell to an electric field of sufficient energy to sensitise the red blood cell so that it is susceptible to disruption by ultrasound.

43. Use of an electroporation apparatus under *in vitro* or *ex vivo* conditions to apply an electric field to a red blood cell for sensitising the red blood cell to disruption by ultrasound.

44. Use of an ultrasound apparatus under *in vitro* or *ex vivo* conditions for the disruption of an electrosensitised red blood cell.

## Patentansprüche

1. Verwendung eines elektrischen Feldes unter *in vitro-* oder *ex vivo-*Bedingungen zum Sensibilisieren eines roten Blutkörperchens gegenüber Ultraschall.

2. Verwendung nach Anspruch 1, wobei das elektrische Feld ausreichend Energie besitzt, um das Blutkörperchen zu elektrosensibilisieren.

3. Verwendung nach Anspruch 1 oder 2, wobei das elektrosensibilisierte rote Blutkörperchen unter Verwendung von Ultraschall gezielt zerstört werden kann.

4. Verwendung nach einem der vorangegangenen Ansprüche, wobei das elektrische Feld unter *in vitro*-Bedingungen eine Stärke von etwa 0,1 kV/cm bis etwa 10 kV/cm hat.

5. Verwendung nach einem der vorangegangenen Ansprüche, wobei die Elektrosensibilisierung die Stufe umfaßt, bei der man einen elektrischen Impuls auf ein rotes Blutkörperchen aufbringt.

6. Verwendung nach Anspruch 5, wobei der elektrische Impuls für zwischen 1 µs und 100 Millisekunden aufgebracht wird.

7. Verwendung nach einem der vorangegangenen Ansprüche, wobei der Ultraschall aus der Gruppe ausgewählt ist, bestehend aus: diagnostischem Ultraschall, therapeutischem Ultraschall und einer Kombination aus diagnostischem und therapeutischem Ultraschall.

8. Verwendung nach einem der vorangegangenen Ansprüche, wobei der Ultraschall unter *in vitro-* oder *ex vivo*-Bedingungen mit einer Leistungshöhe von etwa 0,05 W/cm² bis etwa 100 W/cm² aufgebracht wird.

9. Verwendung nach einem der vorangegangenen Ansprüche, welche weiterhin die Stufe des Beladens des roten Blutkörperchens mit einem Stoff unter *in vitro-* oder *ex vivo*-Bedingungen umfaßt.

10. Verwendung nach Anspruch 9, wobei das Beladen mittels eines Verfahrens durchgeführt wird, ausgewählt aus einer Gruppe, bestehend aus Elektroporation, Sonoporation. Mikroinjektion, Membraninterkalation, Mikropartikelbeschuß, lipidvermittelter Transfektion, Virusinfektion, Osmose, osmotischem Pulsen, Difrusion, Endocytose und Vernetzung mit einem Obeflächenbestandtell des roten Blutkörperchens.

11. Verwendung nach Anspruch 9 oder 10, wobei das Beladen mit dem Stoff im wesentlichen gleichzeitig mit der Sensibilisierung des roten Blutkörperchens erfolgt.

12. Verwendung nach Anspruch 9 oder 10, wobei die Sensibilisierung des roten Blutkörperchens vor dem Beladen mit dem Stoff erfolgt.

13. Verwendung nach Anspruch 9 oder 10, wobei das Beladen mit dem Stoff vor der Sensibilisierung des roten Blutkörperchens erfolgt.

14. Verwendung nach einem der Ansprüche 9 bis 13, wobei der Stoff aus einer Gruppe ausgewählt ist, bestehend aus einem biologisch aktiven Molekül, einem Protein, einem Polypeptid, einem Peptid, einer Nukleinsäure, einem Virus, einem virusartigen Teilchen, einem Nukleotid, einem Ribonukleotid, einem Desoxyribonukleotid, einem modifizierten Desoxyribonukleotid, einer Heteroduplex, einem Nanopartikel, einem synthetischen Analogen eines Nukleotids, einem synthetischen Analogen eines Ribonukleotids, einem modifizierten Nukleotid, einem modifizierten Ribonukleotid, einer Aminosäure, einem Aminosäureanalogen, einer modifizierten Aminosäure, einem modifizierten Aminosäureanalogen, einem Steroid, einem Proteoglycan, einem Lipid, einem Kohlenhydrat und Gemischen, Verschmelzungen, Kombinationen oder Konjugaten der obigen.

15. Verwendung nach einem der Ansprüche 9 bis 14, wobei der Stoff an ein bildgebendes Mittel konjugiert, mit diesem verschmolzen, vermischt oder kombiniert ist.

16. Verwendung nach einem der vorangegangenen Ansprüche, wobei das rote Blutkörperchen PEGyliert ist.

17. Verwendung von Ultraschall zum Zerstören eines elektrosensibilisierten roten Blutkörperchens, wobei sowohl die Elektrosensibilisierung als auch die Zerstörung mittels Ultraschall unter *in vitro-* oder *ex vivo*-Bedingungen durchgeführt werden.

18. Verwendung nach Anspruch 17, wobei das rote Blutkörperchen durch Aussetzen an ein elektrisches Feld, einschließlich eines elektrischen Impulses, elektrosensibilisiert wurde.

19. Verwendung nach Anspruch 17 oder 18, wobei der Ultraschall aus der Gruppe ausgewählt ist, bestehend aus: diagnostischem Ultraschall, therapeutischem Ultraschall und einer Kombination aus diagnostischem und therapeutischem Ultraschall.

20. Verwendung nach Anspruch 17, 18 oder 19, wobei der Ultraschall mit einer Leistungshöhe von etwa 0,05 W/cm² bis etwa 100 W/cm² angelegt wird.

21. Verwendung nach einem der Ansprüche 17 bis 20, wobei das rote Blutkörperchen unter *in vitro-* oder *ex vivo*-Bedingungen mit einem Stoff beladen wird.

22. Verwendung nach Anspruch 21, wobei durch die Ultraschall-Zerstörung des roten Blutkörperchens der Stoff freigesetzt wird.

23. *In vitro-* oder *ex vivo*-Verfahren zum gezielten Zerstören eines roten Blutkörperchens, wobei das Verfahren die folgenden Stufen umfaßt:
(a) Elektrosensibilisieren des roten Blutkörperchens durch Aussetzen des roten Blutkörperchens an ein elektrisches Feld und
(b) Zerstören des roten Blutkörperchens durch Aussetzen des roten Blutkörperchens an Ultraschall.

24. Verfahren nach Anspruch 23, welches weiterhin das Beladen des roten Blutkörperchens mit einem Stoff unter *in vitro-* oder *ex vivo*-Bedingungen umfaßt.

25. *In vitro-* oder *ex vivo*-Verfahren zum gezielten Freisetzen eines Stoffs aus einem roten Blutkörperchen, wobei das Verfahren die folgenden Stufen umfaßt:
(a) Beladen eines roten Blutkörperchens mit einem Stoff,
(b) Elektrosensibilisieren des roten Blutkörperchens und
(c) Veranlassen der Freisetzung des Stoffs aus dem sensibilisierten roten Blutkörperchen durch Anlegen von Ultraschall bei einer Frequenz und mit einer Energie, die ausreichend sind, um eine Zerstörung des roten Blutkörperchens zu verursachen, die jedoch nicht ausreichend sind, um eine Zerstörung nicht sensibilisierter roter Blutkörperchen zu verursachen.

26. *In vitro-* oder *ex vivo*-Verfahren zum Bereitstellen eines Verabreichungsmediums, das für das Verabreichen eines Stoffs an ein Wirbeltier geeignet ist, mit den folgenden Stufen:
(a) Beladen eines roten Blutkörperchens mit einem Stoff und, in einer separaten Stufe,
(b) Aussetzen des roten Blutkörperchens an ein elektrisches Feld, um es gegenüber Zerstörung durch Ultraschall empfindlich zu machen.

27. Verfahren nach Anspruch 25, welches das Aussetzen des roten Blutkörperchens an ein elektrisches Feld umfaßt.

28. Verfahren nach Anspruch 27, wobei das elektrische Feld ausreichend Energie besitzt, um das Blutkörperchen zu elektrosensibilisieren.

29. Verfahren nach einem der Ansprüche 23 bis 28, wobei das elektrische Feld unter *in vitro-*Bedingungen eine Stärke von etwa 0,1 kV/cm bis etwa 10 kV/cm hat.

30. Verfahren nach einem der Ansprüche 23 bis 29, wobei das elektrosensibilisierte rote Blutkörperchen unter Verwendung von Ultraschall gezielt zerstört werden kann.

31. Verfahren nach einem der Ansprüche 23 bis 30, wobei die Elektrosensibilisierung die Stufe umfaßt, bei der man einen elektrischen Impuls auf ein rotes Blutkörperchen aufbringt.

32. Verfahren nach Anspruch 31, wobei der elektrische Impuls für zwischen 1 µs und 100 Millisekunden aufgebracht wird.

33. Verfahren nach einem der Ansprüche 23 bis 32, wobei der Ultraschall aus der Gruppe ausgewählt ist, bestehend aus: diagnostischem Ultraschall, therapeutischem Ultraschall und einer Kombination aus diagnostischem und therapeutischem Ultraschall.

34. Verfahren nach einem der Ansprüche 23 bis 33, wobei der Ultraschall mit einer Leistungshöhe von etwa 0.05 W/cm² bis etwa 100 W/cm² angelegt wird.

35. Verfahren nach einem der Ansprüche 24 bis 34, wobei das Beladen mittels eines Verfahrens durchgeführt wird, ausgewählt aus einer Gruppe, bestehend aus Elektroporation, Sonoporation, Mikroinjektion, Membraninterkalation, Mikroteilchenbeschuß, lipidvermittelter Transfektion, Virusinfektion, Osmose, osmotischem Pulsen, Diffusion, Endocytose und Vernetzung mit einem Oberflächenbestandteil eines roten Blutkörperchens.

36. Verfahren nach einem der Ansprüche 24 bis 35, wobei das Beladen mit dem Stoff im wesentlichen gleichzeitig mit der Sensibilisierung des roten Blutkörperchens stattfindet.

37. Verfahren nach einem der Ansprüche 24 bis 35, wobei die Sensibilisierung des roten Blutkörperchens vor dem Beladen mit dem Stoff erfolgt.

38. Verfahren nach einem der Ansprüche 34 bis 35, wobei das Beladen mit dem Stoff vor der Sensibilisierung des roten Blutkörperchens erfolgt.

39. Verfahren nach einem der Ansprüche 24 bis 38, wobei der Stoff aus einer Gruppe ausgewählt ist, bestehend aus einem biologisch aktiven Molekül, einem Protein, einem Polypeptid, einem Peptid, einer Nukleinsäure, einem Virus, einem virusartigen Teilchen, einem Nukleotid, einem Ribonukleotid, einem Desoxyribonukleotid, einem modifizierten Desoxyribonukleotid, einer Heteroduplex, einem Nanopartikel, einem synthetischen Analogen eines Nukleotids, einem synthetischen Analogen eines Ribonukleotids, einem modifizierten Nukleotid, einem modifizierten Ribonukleotid, einer Aminosäure, einem Aminosäureanalogen, einer modifizierten Aminosäure, einem modifizierten Aminosäureanalogen, einem Steroid, einem Proteoglycan, einem Lipid, einem Kohlenhydrat und Gemischen, Verschmelzungen, Kombinationen oder Konjugaten der obigen.

40. Verfahren nach einem der Ansprüche 24 bis 39, wobei der Stoff an ein bildgebendes Mittel konjugiert, mit diesem verschmolzen, vermischt oder kombiniert ist.

41. Verfahren nach einem der Ansprüche 23 bis 40, wobei das rote Blutkörperchen PEGyliert ist.

42. *In vitro-* oder *ex vivo*-Verfahren zum Elektrosensibilisieren eines roten Blutkörperchens gegenüber Zerstörung durch Ultraschall, wobei das Verfahren die folgenden Stufen umfaßt:
Aussetzen des roten Blutkörperchens an ein elektrisches Feld, welches eine Energie besitzt, die ausreichend ist, um das rote Blutkörperchen so zu sensibilisieren, daß es gegenüber Zerstörung durch Ultraschall empfindlich ist.

43. Verwendung einer Elektroporationsvorrichtung unter *in vitro-* oder *ex vivo*-Bedingungen zum Anlegen eines elektrischen Feldes an ein rotes Blutkörperchen, um das rote Blutkörperchen gegenüber Zerstörung durch Ultraschall zu sensibilisieren.

44. Verwendung einer Ultraschallvorrichtung unter *in vitro-* oder ex *vivo*-Bedingungen zum Zerstören eines elektrosensibilisierten roten Blutkörperchens.

## Revendications

1. Utilisation d'un champ électrique sous des conditions *in vitro* ou *ex vivo* pour sensibiliser aux ultrasons un globule sanguin rouge.

2. Utilisation selon la revendication 1, dans laquelle le champ électrique a une énergie suffisante pour électrosensibiliser la cellule.

3. Utilisation selon la revendication 1 ou 2, dans laquelle le globule sanguin rouge, électrosensibilisé, peut être rompu sélectivement en utilisant un ultrason.

4. Utilisation selon l'une quelconque des revendications précédentes, dans laquelle le champ électrique a une force d'environ 0,1 kV/cm à environ 10 kV/cm sous des conditions *in vitro*.

5. Utilisation selon l'une quelconque des revendications précédentes, dans laquelle l'électrosensibilisation comprend l'étape d'application d'une impulsion électrique à un globule sanguin rouge.

6. Utilisation selon la revendication 5, dans laquelle l'impulsion électrique est appliquée pendant une durée comprise entre 1 µs et 100 millisecondes.

7. Utilisation selon l'une quelconque des revendications précédentes, dans laquelle l'ultrason est sélectionné dans le groupe comprenant : un ultrason diagnostique, un ultrason thérapeutique et une combinaison d'ultrasons diagnostiques et thérapeutiques.

8. Utilisation selon l'une quelconque des revendications précédentes, dans laquelle l'ultrason est appliqué sous des conditions *in vitro* ou ex *vivo* à un niveau de puissance d'environ 0,05 W/cm² à environ 100 W/cm².

9. Utilisation selon l'une quelconque des revendications précédentes, comprenant en outre l'étape de chargement du globule sanguin rouge avec un agent sous des conditions *in vitro* ou *ex vivo*.

10. Utilisation selon la revendication 9, dans laquelle le chargement est effectué par une procédure choisie dans un groupe consistant en une électroporation, une sonoporation, une micro-injection, une intercalation par membrane, un bombardement de microparticules, une transfection à médiation de lipides, une infection virale, une osmose, une pulsion osmotique, une diffusion, une endocytose et une réticulation sur un constituant de surface de globule sanguin rouge.

11. Utilisation selon la revendication 9 ou 10, dans laquelle le chargement de l'agent est sensiblement simultané à la sensibilisation du globule sanguin rouge.

12. Utilisation selon la revendication 9 ou 10, dans laquelle la sensibilisation du globule sanguin rouge précède le chargement de l'agent.

13. Utilisation selon la revendication 9 ou 10, dans laquelle le chargement de l'agent précède la sensibilisation du globule sanguin rouge.

14. Utilisation selon l'une quelconque des revendications 9 à 13, dans laquelle l'agent est choisi dans un groupe constitué d'une molécule biologiquement active, d'une protéine, d'un polypeptide, d'un peptide, d'un acide nucléique, d'un virus, d'une particule analogue à un virus, d'un nucléotide, d'un ribonucléotide, d'un désoxyribonucléotide, d'un désoxyribonucléotide modifié, d'un hétéroduplex, d'une nanoparticule, d'un analogue synthétique d'un nucléotide, d'un analogue synthétique d'un ribonucléotide, d'un nucléotide modifié, d'un ribonucléotide modifié, d'un aminoacide, d'un analogue d'aminoacide, d'un aminoacide modifié, d'un analogue d'aminoacide modifié, d'un stéroïde, d'un protéoglycanne, d'un lipide, d'un glucide, et leurs mélanges, fusions, combinaisons ou conjugaisons.

15. Utilisation selon l'une quelconque des revendications 9 à 14, dans laquelle l'agent est conjugué, fusionné, mélangé ou combiné à un agent de formation d'image.

16. Utilisation selon l'une quelconque des revendications précédentes, dans laquelle le globule sanguin rouge est PEGylé.

17. Utilisation d'un ultrason pour rompre un globule sanguin rouge électrosensibilisé, dans laquelle l'électrosensibilisation et la rupture par ultrason sont exécutées sous des conditions *in vitro* ou *ex vivo.*

18. Utilisation selon la revendication 17, dans laquelle le globule sanguin rouge a été électrosensibilisé en étant exposé à un champ électrique, incluant une impulsion électrique.

19. Utilisation selon la revendication 17 ou 18, dans laquelle l'ultrason est sélectionné dans le groupe constitué de : un ultrason diagnostique, un ultrason thérapeutique et une combinaison d'ultrasons diagnostiques et thérapeutiques.

20. Utilisation selon la revendication 17, 18 ou 19, dans laquelle l'ultrason est appliqué à un niveau de puissance d'environ 0,05 W/cm² à environ 100 W/cm².

21. Utilisation selon l'une quelconque des revendications 17 à 20, dans laquelle le globule sanguin rouge est chargé avec un agent sous des conditions *in vitro* ou *ex vivo*.

22. Utilisation selon la revendication 21, dans laquelle la rupture par ultrason du globule sanguin rouge libère l'agent.

23. Procédé *in vitro* ou *ex vivo* pour rompre sélectivement un globule sanguin rouge, le procédé comprenant les étapes qui consistent :
(a) à électrosensibiliser le globule sanguin rouge en exposant le globule sanguin rouge à un champ électrique ; et
(b) à rompre le globule sanguin rouge en soumettant le globule sanguin rouge à un ultrason.

24. Procédé selon la revendication 23, comprenant en outre le chargement du globule sanguin rouge avec un agent sous des conditions *in vitro* ou *ex vivo*.

25. Procédé *in vitro* ou *ex vivo* pour libérer sélectivement un agent d'un globule sanguin rouge, comprenant les étapes qui consistent :
(a) à charger d'un agent un globule sanguin rouge ;
(b) à électrosensibiliser le globule sanguin rouge ; et
(c) à provoquer la libération de l'agent du globule sanguin rouge sensibilisé en appliquant un ultrason à une fréquence et une énergie suffisantes pour provoquer une rupture du globule sanguin rouge, mais insuffisantes pour provoquer une rupture de globules sanguins rouges non sensibilisés.

26. Procédé *in vitro* ou *ex vivo* pour produire un véhicule d'administration apte à administrer un agent à un être vertébré, comprenant les étapes qui consistent :
(a) à charger d'un agent un globule sanguin rouge ; et, dans une étape séparée,
(b) à exposer le globule sanguin rouge à un champ électrique pour le rendre sujet à une rupture par ultrason.

27. Procédé selon la revendication 25, comprenant le fait de soumettre le globule sanguin rouge à un champ électrique.

28. Procédé selon la revendication 27, dans lequel le champ électrique a une énergie suffisante pour électrosensibiliser le globule.

29. Procédé selon la revendication 23 ou 28, dans lequel le champ électrique a une force d'environ 0,1 kV/cm à environ 10 kV/cm sous des conditions *in vitro.*

30. Procédé selon l'une quelconque des revendications 23 à 29, dans lequel le globule sanguin rouge électrosensibilisé peut être rompu sélectivement en utilisant un ultrason.

31. Procédé selon l'une quelconque des revendications 23 à 30, dans lequel l'électrosensibilisation comprend l'étape d'application d'une impulsion électrique à un globule sanguin rouge.

32. Procédé selon la revendication 31, dans lequel l'impulsion électrique est appliquée pendant une durée de 1 µs à 100 millisecondes.

33. Procédé selon l'une quelconque des revendications 23 à 32, dans lequel l'ultrason est sélectionné dans le groupe constitué de : un ultrason diagnostique, un ultrason thérapeutique et une combinaison d'ultrasons diagnostiques et thérapeutiques.

34. Procédé selon l'une quelconque des revendications 23 à 33, dans lequel l'ultrason est appliqué à un niveau de puissance d'environ 0,05 W/cm² à environ 100 W/cm²,

35. Procédé selon l'une quelconque des revendications 24 à 34, dans lequel le chargement est effectué par une procédure choisie dans un groupe consistant en une électroporation, une sonoporation, une micro-injection, une intercalation par membrane, un bombardement de microparticules, une transfection par médiation de lipides, une infection virale, une osmose, une pulsion osmotique, une diffusion, une endocytose et une réticulation d'un constituant de surface d'un globule sanguin rouge.

36. Procédé selon l'une quelconque des revendications 24 à 35, dans lequel le chargement de l'agent est sensiblement simultané à la sensibilisation du globule sanguin rouge.

37. Procédé selon l'une quelconque des revendications 24 à 35, dans lequel la sensibilisation du globule sanguin rouge précède le chargement de l'agent.

38. Procédé selon l'une quelconque des revendications 24 à 35, dans lequel le chargement de l'agent précède la sensibilisation du globule sanguin rouge.

39. Procédé selon l'une quelconque des revendications 24 à 38, dans lequel l'agent est sélectionné dans un groupe constitué d'une molécule biologiquement active, d'une protéine, d'un polypeptide, d'un peptide, d'un acide nucléique, d'un virus, d'une particule analogue à un virus, d'un nucléotide, d'un ribonucléotide, d'un désoxyribonucléotide, d'un désoxyribonucléotide modifié, d'un hétéroduplex, d'une nanoparticule, d'un analogue synthétique d'un nucléotide, d'un analogue synthétique d'un ribonucléotide, d'un nucléotide modifié, d'un ribonucléotide modifié, d'un aminoacide, d'un analogue d'aminoacide, d'un aminoacide modifié, d'un analogue d'aminoacide modifié, d'un stéroïde, d'un protéoglycanne, d'un lipide, d'un glucide, et leurs mélanges, fusions, combinaisons ou conjugaisons.

40. Procédé selon l'une quelconque des revendications 24 à 39, dans lequel l'agent est conjugué, fusionné, mélangé ou combiné à un agent de formation d'image.

41. Procédé selon l'une quelconque des revendications 23 à 40, dans lequel le globule sanguin rouge est PEGylé.

42. Procédé *in vitro* ou ex *vivo* d'électrosensibilisation d'un globule sanguin rouge pour qu'il soit rompu par ultrason, le procédé comprenant l'étape qui consiste :
à soumettre le globule sanguin rouge à un champ électrique d'énergie suffisante pour sensibiliser le globule sanguin rouge afin qu'il soit sujet à une rupture par ultrason.

43. Utilisation d'un appareil d'électroporation sous des conditions *in vitro* ou ex *vivo* pour appliquer un champ électrique à un globule sanguin rouge afin de sensibiliser le globule sanguin rouge pour une rupture par ultrason.

44. Utilisation d'un appareil à ultrason sous des conditions *in vitro* ou *ex vivo* pour la rupture d'un globule sanguin rouge électrosensibilisé.
